# EUROPÄISCHE PATENTSCHRIFT

(11) **EP 2 199 303 B1**
(45) Veröffentlichungstag und Bekanntmachung des Hinweises auf die Patenterteilung: **05.06.2013**
(21) Anmeldenummer: 08021660.9
(22) Anmeldetag: 12.12.2008
(51) Int. Cl.: C07K 14/20, G01N 33/569

(54) **Polypeptide und Verfahren zur spezifischen Detektion von Antikörpern bei Patienten mit einer Borrelieninfektion**
Polypeptides and method for specific metering of antibodies for patients with a borrelia infection
Polypeptide et procédé de détection spécifique d'anticorps chez des patients ayant une infection de type borrelia

(43) Veröffentlichungstag der Anmeldung: 23.06.2010
(73) Patentinhaber: Euroimmun Medizinische Labordiagnostika AG, 23560 Lübeck (DE)
(72) Erfinder: Komorowski, Lars, 23909 Ratzeburg (DE); Probst, Christian, 23909 Ratzeburg (DE); Janssen, Anthonina, 23847 Schiphorst (DE); Stoecker, Winfried, 23627 Gross Grönau (DE)

(56) Entgegenhaltungen:
- WO-A-97/49812
- RAUER, S. ET AL.: "The Outer Surface Protein C (OspC) from Borrelia burgdorferi can appear as a dimeric molecule in Western Blot" JOURNAL OF SPIROCHETAL AND TICK-BORNE DISEASES, Bd. 3, Nr. 2, Juni 1996 (1996-06), Seiten 105-108, XP002525975
- RAUER SEBASTIAN ET AL: "Enzyme-linked immunosorbent assay using recombinant OspC and internal 14-kDa flagellin fragment for serodiagnosis of early Lyme disease" JOURNAL OF CLINICAL MICROBIOLOGY, Bd. 36, Nr. 4, April 1998 (1998-04), Seiten 857-861, XP002525976 ISSN: 0095-1137
- EARNHART CHRISTOPHER G ET AL: "Disulfide-mediated oligomer formation in Borrelia burgdorferi outer surface protein C, a critical virulence factor and potential Lyme disease vaccine candidate.", CLINICAL AND VACCINE IMMUNOLOGY : CVI JUN 2011 LNKD- PUBMED:21525304, vol. 18, no. 6, June 2011 (2011-06), pages 901-906, ISSN: 1556-679X

## Beschreibung

Die vorliegende Erfindung betrifft von OspC ("*Outer surface Protein C"*) aus Bakterien des Genus *Borrelia* abgeleitete Polypeptide, insbesondere ein Protein nach Anspruch 1, welches ein erstes OspC-Polypeptid umfasst, wobei das erste OspC-Polypeptid über eine Disulfid-Brücke mit einem zweiten OspC-Polypeptid verbunden ist. Die Erfindung betrifft auch ein Verfahren zum Nachweis von Antikörpern gegen OspC nach Anspruch 10 und ein Verfahren zum Nachweis einer Borrelieninfektion nach Anspruch 11, wobei ein erfindungsgemäßes Protein eingesetzt wird, genau wie ein diagnostisches Kit nach Anspruch 13 und einen Impfstoff gegen Borrelien nach Anspruch 15.

### Stand der Technik:

Bakterien des Genus *Borrelia* sind als Erreger mehrerer humaner Erkrankungen, insbesondere als Erreger der Lyme-Borreliose und des Rückfallfiebers, beschrieben. Der Nachweis einer Infektion erfolgt heutzutage meistens über die Bestimmung spezifischer Antikörper gegen die Bakterien in humanen oder tierischen Körperflüssigkeiten. Dabei zeigt das Vorliegen von spezifischen Antikörpern gegen Antigene, die nur in Borrelien vorkommen, eine Infektion mit Borrelien an. Frühe Phasen der Infektion, insbesondere frische Infektionen bis zu vier Wochen nach Erstkontakt, sind durch das Vorliegen von Antikörpern der Klasse IgM, insbesondere gegen die Antigene OspC, p41 (Flagellin) und P39 (BmpA), gekennzeichnet, während späte Phasen, insbesondere abgelaufene, ausgeheilte oder chronisch manifeste Infektionen, mit dem Vorliegen von Antikörpern der Klasse IgG, insbesondere gegen die Antigene VlsE, P83/P100, P58, OspA, p41 (Flagellin), P39, P18 und weitere, einhergehen (Wilske and Fingerle, 2005).

Eine frühe Diagnose der Erkrankung ist besonders wichtig, weil eine Therapie mit Antibiotika in den frühen Stadien erfolgreicher und einfacher ist als in späten Stadien, so ist z.B. in der frühen Phase noch eine orale Therapie mit Antibiotika möglich.

Als wichtigster Marker für eine frühe Phase der Erkrankung gelten gemeinhin Antikörper gegen OspC der Klasse IgM. Bei nativem OspC handelt es sich um ein mit einer Fettsäure acyliertes Membranprotein der Lipoproteinfamilie 6, das in der äußeren Zellmembran verankert ist (Norris *et* al., 1992;Hagman *et* al., 1998). Der Acylrest wird während der Expression parallel zur Abspaltung der Signalsequenz durch Signalpeptidase II an das einzige in der OspC Aminosäureabfolge vorkommende konservierte Cystein angeheftet (Wu and Tokunaga, 1986). Er dient als Verankerung in der lipidhaltigen Zellmembran.

Als wichtigstes Epitop für Reaktionen mit humanen Antikörpern der Klasse IgM innerhalb des OspC gilt allgemeinhin ein konserviertes C-terminales Peptid aus zehn Aminosäureresten, dessen endständige Carboxylgruppe frei zugänglich sein muss (Mathiesen *et al*., 1998a; Mathiesen *et al*., 1998b) wie auch in der PCT-Patentschrift WO 9742221 aufgeführt. Darüber hinaus sind Epitope im wenig konservierten zentralen Bereich des OspC beschrieben (Earnhart *et al*., 2005).

Für den Nachweis der Antikörper werden regelmäßig aus Borrelien aufgereinigte Antigene in immunbiochemischen Tests wie ELISA, Linienblot oder Westernblot verwendet. In den Verfahren werden üblicherweise einzelne oder mehrere Antigene an eine Festphase gebunden, mit der zu analysierenden Körperflüssigkeit in Kontakt gebracht und die gebundenen Antikörper durch ein Reportermolekül nachgewiesen. Derartige Kits werden z.B. von der EUROIMMUN AG als EUROLINE-WB und Anti-Borrelia-plus-VlsE-ELISA vertrieben. Ähnliche Verfahren sind in der Literatur beschrieben (Hansen *et al*., 1988; Cutler and Wright, 1989; Fister *et al*., 1989).

Verfahren, die auf nicht-rekombinanten Präparaten beruhen, sind nur aufwändig zu reproduzieren und deswegen kostenintensiv, weil für die *in vitro*-Kultivierung von Borrelien im Wesentlichen komplexe Kulturmedien verwendet werden, die natürliche, chemisch nicht definierte Bestandteile wie proteinhaltige Serumfraktionen von *Mammalia* und komplexe Proteingemische, z.B. proteolytisch behandelte Muskelextrakte, Hefeextrakte oder Gelatine enthalten. Ein kommerzielles Kit auf dieser Basis ist z.B. von Sigma Aldrich erhältlich (Complete BSK-H). Ähnliche Zusammensetzungen sind mit den Bezeichnungen MKP und BSK II in der Literatur beschrieben (Ruzic-Sabljic and Strle, 2004).

Nachteiligerweise unterliegen die chemisch nicht definierten Bestandteile dieser Medien starken Schwankungen ihrer Zusammensetzungen von Charge zu Charge, bergen das Risiko der Verunreinigung mit Viren oder Mycoplasmen und sind kostenintensiv in der Herstellung. Dementsprechend zeigen *in vitro*-kultivierte Borrelien üblicherweise in Abhängigkeit der jeweils verwendeten Kulturmedien bzw. in Abhängigkeit einzelner Komponenten der jeweils verwendeten Kulturmedien starke Schwankungen ihrer Wachstumsraten und ihrer Genexpressionsmuster. Insbesondere sind hiervon Gene betroffen, die *in vivo* in Abhängigkeit des jeweiligen Wirtsorganismus exprimiert werden (Pollack *et al*., 1993; Yang *et al*., 2001). Dieses gilt insbesondere für das OspC. Des Weiteren haben auch die Kultivierungstemperatur sowie Sauerstoff- und Kohlendioxidkonzentration einen entscheidenen Einfluss auf die Art und Menge der exprimierten Proteine (Seshu *et al*., 2004; Hyde *et al*., 2007). Dadurch lassen sich insbesondere Ergebnisse unterschiedlicher Laboratorien, die Borrelien kultivieren, schlecht miteinander vergleichen.

Verfahren, die auf einzelnen, aus *in vitro*-kultivierten Borrelien gereinigten Antigenen beruhen, sind zudem anfällig für unspezifische Reaktionen, die durch die Anwesenheit von weiteren, nicht genügend abgereicherten Verunreinigungen verursacht werden. Insbesondere in Verfahren, die nur ein Signal generieren, wie etwa ELISA oder Linienblot, werden so regelmäßig falsch positive Ergebnisse erzielt. Demgegenüber weisen Westernblots, die dieses Problem durch die örtliche Auflösung der Signale umgehen, den Nachteil auf, dass sie einen wesentlichen zusätzlichen Aufwand erzeugen, bedingt durch die notwendige Elektrophorese und den Transfer auf eine Membran.

In weiteren Verfahren finden Antigene Verwendung, die durch rekombinante Techniken, z.B. durch heterologe Expression von Antigenen oder Antigenfragmenten in *E. coli*, hergestellt werden, wie z.B. in der europäischen Patentschrift EP 0506868 aufgeführt. Kommerziell erhältliche Varianten sind z.B. EUROLINE-WB und Anti-Borrelia-plus-VlsE-ELISA (IgG) und der recomline Borrelia IgM (Mikrogen). Ähnliche Verfahren sind in der Literatur beschrieben (Hauser *et al*., 1998; Lawrenz *et al*., 1999; Wilske and Fingerle, 2005). Im Allgemeinen weisen rekombinante Antigene den Vorteil auf, dass sie, z.B. durch Fusion mit Aufreinigungspolypeptiden, z.B. dem Polyhistidin-Anhang (= His-Tag), definierter und mit weniger Aufwand aufreinigbar sind als native Antigene.

Die heterologe Expression von OspC inklusive der ihm eigenen Signalsequenz in *E. coli* und folgender Acylierung ist möglich, führt aber zu einer geringen Expressionseffizienz (Fuchs *et al*., 1992). Deswegen werden im Allgemeinen Deletionskonstrukte des OspC mit gesteigerter Expressionseffizienz, bei denen mindestens die Signalsequenz fehlt, eingesetzt. Dabei werden im Stand der Technik mindestens die ersten 19 Aminosäuren des OspC, inklusive des in der OspC Aminosäureabfolge vorkommenden Cysteins, deletiert (Fuchs *et al*., 1992; Wilske *et al*., 1993; Wilske *et al*., 1995; Eicken *et al*., 2001; Kumaran *et al*., 2001) und in manchen Fällen durch N-terminale Fusion mit heterologen Polypeptiden, z.B. dem His-Tag, ergänzt.

Solche N-terminal verkürzten Varianten wurden auch zur Aufklärung der Raumstruktur des OspC verwendet (Kumaran *et al*., 2001; Eicken *et al*., 2001). Dazu wurden die ersten 31 (Eicken *et al*., 2001) oder 37 (Kumaran *et al*., 2001) Aminosäurereste des OspC in den rekombinanten OspC-Varianten deletiert, um insbesondere eine geeignete Menge und Reinheit des OspC zu erreichen. Aus den beiden exemplarischen Raumstrukturen geht hervor, dass rekombinantes OspC zur Dimerisierung neigt. Diese Dimerisierung erfolgt aufgrund von ionischen Wechselwirkungen und Wasserstoffbrückenbindungen, nicht jedoch durch eine kovalente Verknüpfung.

Es gibt auch Versuche, acylierte Varianten des OspC durch Vorschalten einer heterologen Signalsequenz rekombinant herzustellen, wie z.B. in der europäischen Patentschrift EP 1741718 aufgeführt. Durch Anheftung der Fettsäure sollen solche OspC-Varianten eine höhere *in vivo*-Immunogenität als die bis dahin beschriebenen N-terminal deletierten oder modifizierten OspC-Varianten besitzen.

Rauer et al., 1996, Journal of spirochetal and tick-borne diseases 3, 105-108, beschreibt eine Untersuchung von nativem und rekombinanten OspC aus den Stämmen GrHo und PKo. Bei Zellextraktion unter nicht reduzierenden Bedingungen kann sich eine Disulfidbrücke an einem Cystein ausbilden. In Rauer et al., 1998, Journal of clinical microbiology 36, 857-861, wird ein ELISA zur Bestimmung von humanem IgM, gerichtet gegen Antigene aus Borrelien, unter Verwendung eines rekombinanten internen Fragments von Flagellin in Kombination mit rekombinantem OspC aus dem Stamm GeHo offenbart.

In WO 97/49812 A1 wird ein Verfahren zur Reinigung von rekombinantem, nicht lipidiertem, Osp-Protein beschrieben.

Die bisher für die Diagnostik verwendeten rekombinanten Varianten des OspC weisen im Allgemeinen den Nachteil auf, dass sie sich von dem aus *in vitro*-kultivierten Borrelien gereinigten nativen OspC unterscheiden, weil sie nicht oder anders posttranslational modifiziert sind und die Anwesenheit konformationeller Epitope bzw. die Zugänglichkeit von Epitopen allgemein nicht vergleichbar bzw. nicht gewährleistet ist. Ausdruck dieser Nachteile ist die regelmäßig in der Literatur beschriebene geringere spezifische Reaktivität bisher verwendeter rekombinanter OspC-Varianten gegenüber nicht-rekombinanten Präparaten. Sie schlägt sich in hohen Raten falsch positiver Ergebnisse in solchen diagnostischen Verfahren nieder.

Die Entwicklung von rekombinanten OspC-Varianten, die sich bei einfacherer und definierterer Darstellung gegenüber den nativen Antigenen in diagnostischen Verfahren einsetzen lassen und gleichzeitig nicht zu den für bisherige rekombianten OspC-Varianten beschriebenen Nachteilen führen, ist daher erforderlich, um die Herstellung solcher *in* vitro-Diagnostika bzw. die Durchführung solcher diagnostischer Verfahren zu erleichtern bzw. Ergebnisse verschiedener Laboratorien zu standardisieren.

### Beschreibung der Erfindung:

Überraschenderweise wurde nun festgestellt, dass sich die beschriebenen Probleme durch die Erfindung, insbesondere den Gegenstand der Ansprüche, lösen lassen.

Gegenstand der Erfindung ist ein Protein nach Anspruch 1, das ein erstes OspC-Polypeptid umfasst, wobei das erste OspC-Polypeptid kovalent mit einem zweiten OspC-Polypeptid verbunden ist. Das erste OspC-Polypeptidist über eine Disulfid-Brücke mit einem zweiten OspC-Polypeptid verbunden, wobei die Disulfid-Brücke durch Verbindung von einem Cystein entsteht, das nicht mehr als 100 Aminosäurepositionen vom N-Terminus des OspC-Polypeptids entfernt ist.

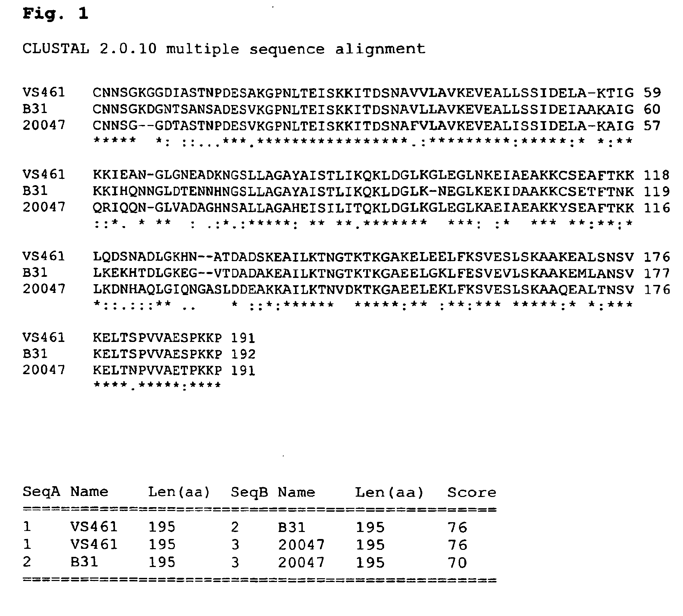

Ein OspC-Polypeptid im Sinne der Erfindung, insbesondere das erste und/oder zweite OspC-Polypeptid, weist eine Aminosäureidentität von mindestens 70% mit SEQ ID NO: 3, SEQ ID NO: 6 oder SEQ ID NO: 9 auf. Es wird (in diesem homologen Bereich) spezifisch von Antikörper gegen OspC aus Borrelien erkannt. Bevorzugt beträgt die Aminosäureidentität mindestens 80%, mindestens 90%, mindestens 95% oder mindestens 99% zu einer oder mehreren der Sequenzen nach SEQ ID NO: 3, SEQ ID NO: 6 oder SEQ ID NO: 9. Diese Sequenzen entsprechen den nativen OspC Sequenzen aus den drei Typ-Stämmen der Borrelien Genospezies *Borrelia afzelii* (VS461), *Borrelia garinii* (20047) und *Borrelia burgdorferi* (B31). Die Aminosäureidentität zwischen diesen Stämmen beträgt 70% bzw. 76% (siehe Fig. 1). Die Aminosäureidentität wird bevorzugt mit dem Programm CLUSTAL 2.0.10 mit Standardeinstellungen ermittelt. Im Rahmen der Erfindung kann insbesondere eine OspC-Sequenz aus einem anderen Borrelienstamm, z.B. PKo, oder eine Variante davon eingesetzt werden, entscheidend ist die spezifische Bindung durch Antikörper gegen OspC aus Borrelien.

Eine spezifische Erkennung von Antikörpern gegen OspC aus Borrelien bedeutet im Rahmen der Erfindung, dass Antikörper gegen OspC aus Borrelien unter dazu geeigneten Bedingungen, die dem Fachmann bekannt und etwa in den Beispielen beschrieben sind, an das Polypeptid bzw. Protein binden, nicht aber an ein anderes, nicht verwandtes Protein. Als Antikörper gegen OspC aus Borrelien kann etwa Serum von Patienten mit gesicherter früher Phase einer Borrelieninfektion eingesetzt werden, aber auch Antikörper, welche z.B. durch Immunisierung von Mäusen oder Kaninchen mit nativem aufgereinigten OspC (z.B. EUROIMMUN) hergestellt wurden, insbesondere polyklonale Antikörper.

Borrelien umfassen alle Bakterien des Genus Borrelia, beispielhaft sind *B. burgdorferii, B. garinii* und *B. afzelii* genannt.

Ein OspC-Polypeptid im Sinne der Erfindung, insbesondere das erste und/oder zweite OspC-Polypeptid, kann mindestens ein Epitop umfassen, welches spezifisch von Antikörper gegen OspC aus Borrelien erkannt wird, wobei das Epitop mindestens eine Teilsequenz von 10 fortlaufenden Aminosäuren aus SEQ ID NO: 3, SEQ ID NO: 6 oder SEQ ID NO: 9 umfasst, insbesondere die 10 C-terminalen Aminosäuren dieser Sequenzen. Bevorzugt umfasst die Teilsequenz das in Mathiesen *et al*., 1998a; Mathiesen *et al*., 1998b als immunodominant beschriebene Epitop. Insbesondere umfasst das bzw. die OspC-Polypeptide C-terminal eine der Aminosäuresequenzen nach SEQ ID NO: 2, SEQ ID NO: 5, oder SEQ ID NO: 8. Es ist bevorzugt, dass das bzw. die OspC-Polypeptide am C-Terminus mit der Sequenz des nativen OspC enden, hier also keine weiteren Aminosäuren enthalten sind, welche die Erkennung des C-terminalen Epitops behindern könnten.

Alternativ oder zusätzlich können ein oder mehrere weitere Epitope, z.B. wie von Earnhart *et al*., 2005 beschrieben, in dem OspC-Polypeptid vorliegen.

Bevorzugt ist das Epitop bzw. sind die Epitope in weitere Sequenzen aus OspC entsprechend einer Wildtyp-Sequenz von OspC aus Borrelien eingebettet. Bevorzugt hat die Teilsequenz von fortlaufenden Aminosäuren aus SEQ ID NO: 3, SEQ ID NO: 6 oder SEQ ID NO: 9 eine Länge von mindestens 15, mindestens 20, mindestens 25, mindestens 30, mindestens 50, mindestens 100, mindestens 150 oder mindestens 190 Aminosäuren. Bevorzugt handelt es sich dabei um die C-terminalen Aminosäuren aus SEQ ID NO: 3, SEQ ID NO: 6 oder SEQ ID NO: 9, von Homologen aus anderen Borrelienstämmen oder Homologen mit einer Sequenzidentität von mindestens 70%, mindestens 80%, mindestens 90%, mindestens 95% oder mindestens 99% mit SEQ ID NO: 3, SEQ ID NO: 6 oder SEQ ID NO: 9.

Die erfindungsgemäßen OspC-Polypeptide weisen keine Signalsequenzen für eine Acylierung auf. Im Stand der Technik (EP 1741718) exprimierte Polypeptide, welche wie das Wildtyp-Polypeptid an dem am weitesten N-terminal gelegenen Cystein acyliert sein sollten, wurden mit einer heterologen Signalsequenz exprimiert. Im Gegensatz dazu enthält das erfindungsgemäße Polypeptid weder eine homologe Signalsequenz noch eine heterologe Signalsequenz. Signalsequenzen, die zu einer Acylierung führen, umfassen üblicherweise eine Braun sche Signalsequenz, die durch Signalpeptidase II erkannt wird. Solche Signalsequenzen können z.B. die Sequenz L-I-A-C (wie OspA oder OspB) oder L-X-Y-C (wobei X und Y kleine neutrale Aminosäuren sind) umfassen (Fuchs *et al*., 1992). Bevorzugt wurde das erfindungsgemäße Polypeptid auch nicht mit einer Acylierungssequenz exprimiert und diese dann abgespalten. Das erfindungsgemäße OspC-Polypeptid ist damit auch nicht acyliert. Das Cystein, an dem die Acylierung im Wildtyp-OspC stattfinden würde, ist in einer Ausführungsform der Erfindung das Cystein, an dem die Disulfidbrücke zu einem weiteren OspC-Polypeptid ausgebildet ist.

Alternativ oder zusätzlich kann eine Disulfidbrücke auch an einem anderen Cystein ausgebildet sein. Bevorzugt entsteht die Disulfidbrücke durch Verbindung von einem Cystein, das nicht mehr als etwa 100, bevorzugt nicht mehr als etwa 50 oder nicht mehr als etwa 30 Aminosäurepositionen vom N-Terminus des OspC-Polypeptids entfernt ist. Insbesondere kann die Disulfidbrücke an einem Cystein ausgebildet werden, welches den N-Terminus des Polypeptids bildet, oder 1, 2, 3, 4, 5, 6, 7, 8, 9, 10, 11, 12, 13, 14, 15, 16, 17, 18, 19, 20, 21, 22, 23, 24, 25, 26, 27, 28, 29 oder 30 Aminosäuren vom N-Terminus entfernt ist.

Um die Aufreinigung des erfindungsgemäßen Proteins zu erleichtern, enthält das OspC-Polypeptid/die OspC-Polypeptide in einer Ausführungsform am N-Terminus, insbesondere N-terminal von dem Cystein, an dem sich die Disulfidbrücke ausbildet, einen His-Tag, welcher z.B. die Sequenz entsprechend den ersten 12 Aminosäuren von SEQ ID NO: 1 aufweisen kann. Statt 8 können auch z.B. 6 oder 10 Histidine eingesetzt werden. Auch andere im Stand der Technik bekannte, die Aufreinigung oder den Nachweis des Proteins erleichternde Aminosäuresequenzen, z.B. ein Flag-Tag, können hier eingebaut werden. Solche Sequenzen sollten die Erkennung durch Antikörper gegen OspC aus Borrelien nicht verhindern. Unter dieser Voraussetzung ist auch an anderer Lokalisation in dem Molekül die Verbindung oder der Einbau eines Reportermoleküls möglich. Das Reportermolekül kann z.B. ausgewählt sein aus einer Gruppe umfassend His-Tag, Flag-Tag, eine Fluoreszenzmarkierung, Biotin und Streptavidin.

In einer Ausführungsform umfasst das erste OspC-Polypeptid N-terminal einen His-Tag, der an ein Cystein angrenzt, welches mit dem zweiten OspC-Polypeptid in dem erfindungsgemäßen Protein über eine Disulfidbrücke verbunden ist, und darauffolgend in der Polypeptidkette des ersten OspC-Polypeptids die weitere Sequenz eines OspC-Polypeptids aus einem Borrelienstamm, z.B. B. burgdorferii, B. afzelii oder B. garinii, wobei diese Sequenz den C-Terminus des Polypeptids bildet. Insbesondere kann das OspC-Polypeptid aus diesen Sequenzen bestehen. Das zweite (und gegebenenfalls weitere) OspC-Polypeptid umfasst bevorzugt die gleichen Sequenzbestandteile His-Tag - Cys - OspC-Sequenz) bzw. besteht daraus.

In einer bevorzugten Ausführungsform weist das erste und/oder das zweite OspC-Polypeptid eine Sequenz nach SEQ ID NO: 2, 5 oder 8 auf.

Im Rahmen der vorliegenden Erfindung wird "ein" im Allgemeinen nicht als Zahlwort gebraucht, sondern damit kann eine unbestimmte Anzahl beschrieben werden. Sofern nicht anders beschrieben, kann es also auch für zwei oder mehrere stehen. Insbesondere wird unter einem OspC-Polypeptid im Sinne der Erfindung das erste und/oder das zweite OspC-Polypeptid eines erfindungsgemäßen Proteins verstanden. Dies schließt die Anwesenheit weiterer OspC-Polypeptide nicht aus, welche bevorzugt die gleiche Struktur wie die ersten OspC-Polypeptide aufweisen.

Bevorzugt sind die erfindungsgemäßen Proteine Dimere von zwei OspC-Polypeptiden. Auch eine Multimerisierung mit weiteren OspC-Polypeptiden oder anderen Molekülen (z.B. über zusätzliche Disulfidbrücken) ist grundsätzlich möglich, solange dies die Erkennung durch Antikörper gegen OspC aus Borrelien nicht verhindert oder einschränkt. Es wurde im Rahmen der Erfindung gezeigt, dass durch die Disulfidbrücke ein Homodimer von zwei gleichen OspC-Polypeptiden oder ein Heterodimer unterschiedlicher OspC-Polypeptide entstehen kann. Damit ist es z.B. möglich, mit einem erfindungsgemäßen Protein den Nachweis von Antikörpern gegen mehrere Borrelien-Stämme zu führen oder einen Impfstoff gegen mehrere Borrelien-Stämme herzustellen.

In Bezug auf die vorliegende Erfindung wird Protein im allgemeinen für Multimere von OspC-Polypeptiden verwendet und Polypeptid für einzelne OspC-Polypeptidketten, beide Begriffe können sich aber, was für den Fachmann jeweils aus dem Zusammenhang ersichtlich ist, auch auf Proteine, Polypeptide oder Peptide nach allgemeinen wissenschaftlichen Sprachgebrauch beziehen, wobei zwischen unterschiedlicher Größe nicht differenziert wird.

Im Rahmen der Erfindung wurde gezeigt, dass die Disulfidbrückenbindung direkt zwischen den OspC-Polypeptiden die Erkennung durch Antikörper gegen OspC aus Borrelien deutlich verbessert. Damit geht einher, dass sich die Erkennung des Proteins durch Antikörper gegen OspC aus Borrelien durch in Kontakt Bringen mit mindestens einem Reagenz verringert, das die Disulfidbrücke zerstört. Insbesondere ist ein solches Reagenz ausgewählt aus einer Gruppe umfassend Thiol-haltige Reagenz oder einer Kombination aus einem Thiol-haltigen Reagenz und einem Alkylhalogenid. Solches Reagenzien beinhalten z.B. Reduktionsmittel, wie Dithiothreitol oder Mercaptoethanol, gegebenenfalls in Kombination mit einem Alkylierungsmittel wie Iodacetamid. Insbesondere kann man zunächst ein Reduktionsmittel und dann ein Alkylhalogenid einsetzen.

Ein erfindungsgemäßes Protein kann z.B. in Bakterienzellen (z.B. *E*. *coli, z.B. E. coli* RosettaBlue(DE3)pLacI (Stratagene)), Insektenzellen (z.B. SF6-Zellen), Hefezellen (z.B. *S. cerevisiae*) oder Zellen von Vertebraten (z.B. CHO-Zellen) hergestellt werden. Die Erfindung umfasst ein Verfahren zur Herstellung des erfindungsgemäßen Proteins, bei dem dieses rekombinant exprimiert und aufgereinigt wird. Eine Aufreinigung ist z.B. über eine Affinitätschromatographie möglich, wobei etwa Antikörper gegen OspC aus Borrelien oder - bei Verwendung z.B. eines His-Tags - eine Nickel-Affinitätschromatographie verwendet werden kann. Bevorzugt werden bei der Expression und/oder Aufreinigung nicht-reduzierende Bedingungen verwendet. Bevorzugt wird bei der Aufreinigung ein pH von 7-9, ein pH von 7,5 - 8,5, insbesondere pH 8 verwendet. Bei einer Affinitätschromatographie kann z.B. die aufzureinigende Mischung mit TNI10-Puffer aufgetragen und gewaschen und das Protein mit TNI150-Puffer eluiert werden. Andere Herstellungs- und Aufreinigungsverfahren, welche die Ausbildung der Disulfidbrücke nicht behindern, sind im Stand der Technik bekannt.

In einem Aspekt richtet sich die vorliegende Erfindung auf ein Verfahren zum Nachweis von Antikörpern gegen ein erfindungsgemäßes Protein, bei dem man eine biologische Probe mit dem erfindungsgemäßen Protein in Kontakt bringt und die Bindung von Antikörpern, welche gegebenenfalls in der Probe vorliegen können, an das Protein nachweist.

Insbesondere wird ein Verfahren zur Diagnose einer Borrelieninfektion offenbart, bei dem man eine biologische Probe eines Patienten mit einem erfindungsgemäßen Protein in Kontakt bringt und die Bindung von Antikörpern, welche gegebenenfalls in der Probe vorliegen können, an das Protein nachweist, wobei ein Nachweis der Bindung von Antikörpern auf eine Borrelieninfektion hinweist.

Gegenstand der Erfindung ist auch die Verwendung eines erfindungsgemäßen Proteins zum Nachweis von Antikörpern gegen OspC von Borrelien und zur Diagnose einer Borrelieninfektion. Die nachgewiesenen Antikörper sind bevorzugt Antikörper vom Typ IgM, wodurch eine Borrelieninfektion in einer frühen Phase nachgewiesen wird.

Die Bindung der Antikörper kann z.B. mit einem Immunfluoreszenztest, ELISA, Lumineszenztest, Westernblot, Linienblot oder Dot Blot nachgewiesen werden. Als biologische Probe kann z.B. eine Körperflüssigkeit eines Patienten, insbesondere Blut, Serum, Plasma, Speichel, Urin, oder Liquor eingesetzt werden. Bevorzugt handelt es sich bei dem Patienten um einen menschlichen oder tierischen Patienten, bei dem etwa auf eine Infektion mit Borrelien getestet werden soll.

Gegenstand der Erfindung ist auch ein Kit zur Diagnose von Borrelieninfektionen, welches ein erfindungsgemäßes Protein umfasst. Ein solches Kit kann ferner Antikörper gegen OspC aus Borrelien und/oder für den Nachweis geeignete Puffer und/oder Reagenzien umfassen, wie z.B. einen Antikörper, der humanes IgM erkennt, optional mit einem zum Nachweis geeigneten Reagenz markiert (z.B. einem Fluoreszenzfarbstoff wie FITC oder PE, einem Enzym wie Alkalische Phosphatase oder Meerettich-Peroxidase oder Biotin). Es kann sich z.B. um ein Kit für Immunfluoreszenztest, ELISA, Lumineszenztest, Westernblot, Linienblot oder Dot Blot handeln.

Gegenstand der Erfindung ist auch die Verwendung eines erfindungsgemäßen Proteins zur Herstellung eines Impfstoffs gegen eine Borrelieninfektion, sowie ein Impfstoff gegen eine Borrelieninfektion, welcher ein erfindungsgemäßes Protein umfasst. Ein solcher Impfstoff kann geeignete Hilfsstoffe, Puffer und/oder Adjuvantien umfassen. Es kann sich um einen Kombinationsimpfstoff handeln, der etwa gegen zwei, drei oder mehr Borrelienstämme gerichtet sein kann. Dies kann durch den Einsatz von heteromeren OspC-Proteinen erreicht werden, aber auch z.B. durch Mischung von verschiedenen Homodimeren. Ein solcher Impfstoff kann z.B. zur subkutanen, intramuskulären, intravenösen oder oralen Verabreichung formuliert sein. Eine mehrfache Verabreichung, z.B. zweimal, dreimal oder viermal (etwa Tag 1, 14, 28, 42) kann sinnvoll sein, um die Bildung von Antikörpern zu verbessern.

Die vorliegende Erfindung ermöglicht erstmalig die Bestimmung OspC-spezifischer Antikörper mit Hilfe rekombinanter OspC-Varianten, die diagnostisch gleichwertige Ergebnisse wie bei der Verwendung nicht-rekombinanter OspC-Varianten liefern. Gleichzeitig ermöglicht der Einsatz der neuen Disulfid-verbrückten rekombinanten OspC-Varianten eine erhebliche Reduktion des Aufwands bei der Herstellung der Antigene gegenüber den bisher als diagnostisch am kompetentesten geltenden nativen OspC-Varianten.

Es wurde gezeigt, dass die OspC-Varianten gemäß SEQ ID NO 1, SEQ ID NO 2 (abgeleitet von maturem OspC gemäß SEQ ID NO 3 aus *B. afzelii* VS461 = OspC_{VS461}, Genbank Accession No. D49379), SEQ ID NO 4, SEQ ID NO 5 (abgeleitet von OspC gemäß SEQ ID NO 6 aus *B. garinii* 20047 = OspC₂₀₀₄₇, Genbank Accession No. L42900), SEQ ID NO 7 und SEQ ID NO 8 (abgeleitet von OspC gemäß SEQ ID NO 9 aus *B. burgdorferi* B31 = OspC_{B31}, Genbank Accession No. U01894) in großen Mengen in *E. coli* exprimierbar und mit hoher Reinheit darstellbar sind. Dabei zeigte sich kein Unterschied im Aufwand der gentechnischen Arbeiten, Expressionsrate, Aufwand der Darstellung oder Reinheit zwischen den sich jeweils nur um ein Cystein unterscheidenden Varianten gemäß SEQ ID NO 1 und SEQ ID NO 2 (abgeleitet von OspC_{VS461}), SEQ ID NO 4 und SEQ ID NO 5 (abgeleitet von OspC₂₀₀₄₇) und SEQ ID NO 7 und SEQ ID NO 8 (abgeleitet von OspC_{B31}).

Darüber hinaus wurde exemplarisch gezeigt, dass die diagnostische Genauigkeit eines Verfahrens zur Bestimmung von Antikörpern der Klasse IgM unter Verwendung der von OspC_{VS461} abgeleiteten Variante gemäß SEQ ID NO 2 überraschenderweise gleichwertig zu einem etablierten Verfahren unter Verwendung von nicht-rekombinantem OspC des Stammes VS461 ist. Weiterhin wurde gezeigt, dass die diagnostische Genauigkeit dieser beiden Verfahren größer ist als bei Verfahren, die die von OspC_{VS461} abgeleitete Variante gemäß SEQ ID NO 1 oder gemäß der wissenschaftlichen Literatur deletierten rekombinanten OspC-Varianten verwenden.

Des Weiteren wurde exemplarisch gezeigt, dass die von OspC_{VS461} abgeleitete Variante gemäß SEQ ID NO 2 nach biochemischer Aufreinigung über eine Disulfidbrücke dimerisiert vorliegt und überraschenderweise eine höhere spezifische Reaktivität als ihr Monomer, ihr am Cystein acetyliertes Monomer und das analoge Monomer gemäß SEQ ID NO 1 aufweist.

Des Weiteren wurde exemplarisch gezeigt, dass sich durch Vermischung der Varianten gemäß SEQ ID NO 2 (abgeleitet von OspC_{VS461}) und SEQ ID NO 5 (abgeleitet von OspC₂₀₀₄₇), gefolgt von deren Reduktion und anschließender Oxidation Disulfid-verbrückte Heterodimere herstellen lassen, deren Einsetzbarkeit grundsätzlich gleichwertig zu den Disulfid-verbrückten Homodimeren ist, die aber den Vorteil haben, mehrere unterschiedliche Spezies-spezifische Antikörper erfassen zu können, d.h. Antikörper, die sich ausschließlich gegen OspC aus *B. afzelii-*Stämmen oder ausschließlich gegen OspC aus *B. garinii*-Stämmen richten. Dem Fachmann erschließen sich Mittel und Verfahren, um Heterodimere von Homodimeren zu trennen, z.B. durch isoelektrische Fokussierung. Eine solche Trennung ist aber nicht notwendig, da auch Mischungen von Homodimeren und Heterodimeren verwendet werden können.

Des Weiteren wurde exemplarisch gezeigt, dass Mäuse überraschenderweise nach Immunisierung mit der über Disulfbrücken dimerisierten OspC-Variante gemäß SEQ ID NO 2 (abgeleitet von OspC_{VS461}) einen höheren Titer von Antikörpern gegen Borrelien aufweisen als nach einer Immunisierung mit der gleichen Menge der monomeren OspC-Variante gemäß SEQ ID NO 1. Daraus kann auf eine höhere Effektivität von auf den erfindungsgemäßen Polypeptiden basierenden Impfstoffen geschlossen werden. Die Ergebnisse sind auf die anderen Varianten, auf andere Tiere und auf Menschen übertragbar.

Die folgenden Beispiele stellen den Gegenstand der Erfindung exemplarisch dar, sollen die Erfindung aber nicht einschränken.

### Legende

Figur 1 zeigt ein Alignment von maturem OspC aus *B. afzelii* VS461 = OspC_{VS461}, aus *B*. *garinii,* 20047 = OspC₂₀₀₄₇ und aus *B*. *burgdorferi* B31 = OspC_{B31}, ermittelt mit CLUSTAL 2.0.10 mit Standardeinstellungen. Die Tabelle gibt den Grad der Aminosäureidentität an.

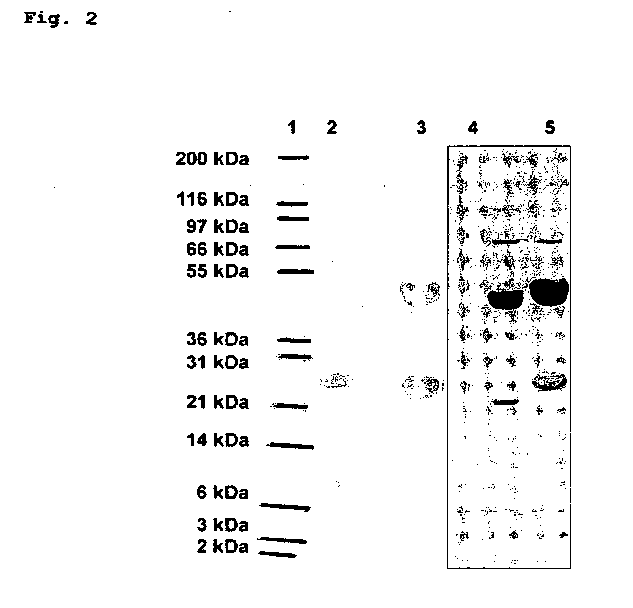

Figur 2 zeigt exemplarisch einen Westernblot mit den nichtreduzierten Varianten His-m-OspC_{VS461} und His-m-Cys-OspC_{VS461} nach Färbung mit Ponceaus S (Spur 1-3) sowie nach Inkubation mit einem Serum eines Patienten mit einer Borreliose im frühen Stadium (1:200 Verdünnung) und Nachweis gebundener IgM-Antikörper (Spur 4 & 5). Spur 1: Größenmarker Mark12 (Invitrogen), Spur 2 & 4: 15 µg His-m-OspC_{VS461}, Spur 3 & 5: 25 µg His-m-Cys-OspC_{VS461}.

### Beispiele

### Beispiel 1: Klonierung von Vektoren zur Expression von OspC in E. coli

OspC ist in Borrelien auf dem Plasmid B kodiert (Nomenklatur aufgrund des vollständigen *B. burgdorferi* Genoms, Leserahmen BB_B19). Als Template zur PCR-Amplifikation der für sechs OspC-Varianten kodierenden Genbereiche gemäß SEQ ID NO 10 bis 15 wird Gesamt-DNA aus *B. afzelii* Stamm VS461, *B. garinii* Stamm 20047 und *B*. *burgdorferi* Stamm B31 verwendet. Für die Amplifikation der einzelnen Varianten werden die Primerkombinationen gemäß SEQ ID NO 16 und SEQ ID NO 17 (His-m-OspC_{VS461}), SEQ ID NO 18 und SEQ ID NO 17 (His-Cys-m-OspC_{VS461}), SEQ ID NO 19 und SEQ ID NO 20 (His-m-OspC₂₀₀₄₇), SEQ ID NO 21 und SEQ ID NO 20 (His-Cys-m-OspC₂₀₀₄₇), SEQ ID NO 22 und SEQ ID NO 17 (His-m-OspC_{B31}) und SEQ ID NO 23 und SEQ ID NO 17 (His-Cys-m-OspC₃₁) eingesetzt. Durch die PCR werden in die Amplifikate die notwendigen Restriktionsschnittstellen jeweils am 5'- bzw. 3'-Ende des kodierenden Stranges integriert.

Die PCR wird unter Verwendung des "High Fidelity PCR Enzyme Mix" (Fermentas) entsprechend den Herstellerangaben im 50 µl Maßstab durchgeführt. Es werden 30 Reaktionszyklen, zusammengesetzt aus den Schritten 1 bis 3, durchgeführt:

| | | |
|---|---|---|
| Schritt 1 | 30 Sekunden | 94°C |
| Schritt 2 | 45 Sekunden | 56°C |
| Schritt 3 | 30 Sekunden | 72°C. |

Die PCR-Fragmente werden anschließend unter Verwendung des NucleoSpin® Extract II-Aufreinigungssystems (MACHEREY-NAGEL GmbH & Co. KG) entsprechend den Angaben des Herstellers aufgereinigt und in jeweils 50 µl 5 mM Tris-HCl pH 8,5 aufgenommen. Anschließend werden die Fragmente durch Restriktionsendonukleasen an den Enden gespalten. Die Restriktionsendonukleasen werden von der Firma Fermentas bezogen und entsprechend den Angaben des Herstellers eingesetzt.

Die Amplifikate werden folgendermaßen mit Restriktionsendonukleasen behandelt:
His-m-OspC_{VS461}: NcoI und XhoI,
His-Cys-m-OspC_{VS461}: Esp3I und XhoI,
His-m-OspC₂₀₀₄₇: NcoI und XhoI,
His-Cys-m-OspC₂₀₀₄₇: Esp3I und XhoI,
His-m-OspC_{B31}: NcoI und XhoI,
His-Cys-m-OspC_{B31}: PagI und XhoI.

Die PCR-Fragmente werden anschließend unter Verwendung des NucleoSpin® Extract II-Aufreinigungssystems entsprechend den Angaben des Herstellers aufgereinigt und in jeweils 50 µl 5 mM Tris-HCl pH 8,5 aufgenommen.

Die enzymatisch geschnittenen und aufgereinigten PCR-Fragmente werden anschließend in NcoI/XhoI geschnittenes Plasmid gemäß SEQ ID NO 24 durch Ligation integriert. Zur Ligation wird das Rapid DNA Ligation Kit der Firma Fermentas entsprechend den Angaben des Herstellers eingesetzt. Der Ligationsansatz wird in *E.coli* RosettaBlue(DE3)pLacI (Stratagene) transformiert.

Positive Klone werden aufgrund der Kanamycin/Chloramphenicol [50/34 µg/ml] Resistenz selektiert. Aus diesen Klonen werden die enthaltenen Plasmide isoliert und durch Restriktionsanalyse und DNA-Sequenzierung überprüft. Korrekte Plasmide werden für die Durchführung der Expression ausgewählt, in 20 ml antibiotikahaltigem LB-Medium inokuliert und bei 37°C bis zum Erreichen einer OD600 (d=1 cm) von 0,6 inkubiert. Durch Zugabe von IPTG (Endkonzentration: 1 mM) wird die Proteinexpression induziert und anschließend für weitere 3 Stunden bei 37°C inkubiert. Nach dieser Zeit werden die Zellen durch Zentrifugation bei 2.200 x g für 10 Minuten sedimentiert, in 20 ml PBS resuspendiert, erneut zentrifugiert und schließlich in 1 ml PBS resuspendiert.

Der Zellaufschluss erfolgt durch Zugabe von einem Drittel Volumen 4 x NuPAGE-LDS-Probenpuffer (Invitrogen), enthaltend 141 mM Tris-Cl pH 8,5, 2% (w/v) Lithiumdodecylsulfat, 10% (w/v) Glycerol, 0,51 mM EDTA, 0,22 mM SERVA Blue G250, gefolgt von einer 10 minütigen Inkubation bei 70°C. Chromosomale DNA wird anschließend durch Ultraschallbehandlung (Branson Sonifier, Stufe 7, MicroTip) fragmentiert.

Das Zelllysat wird nach der Trennung durch SDS-PAGE auf eine Nitrocellulose-Membran transferiert. Anschließend werden unabgesättigte Positionen der Membran durch eine 15 minütige Inkubation mit "Universalpuffer" (EUROIMMUN) mit 3% (w/v) Milchpulver blockiert. Anschließend wird 1 Stunde mit einem gegen den His-Tag gerichteten und 1:2000 in "Universalpuffer" mit 3% (w/v) Milchpulver verdünnten monoklonalen Antikörper aus der Maus (Merck Biosciences GmbH) inkubiert. Dann wird dreimal jeweils 5 Minuten mit "Universalpuffer" gewaschen. In einem zweiten Inkubationsschritt reagieren die im positiven Fall an die Proteine gebundenen Antikörper mit einer 1:2000 in "Universalpuffer" verdünnten Konjugat-Lösung, die als Konjugat einen alkalische Phosphatase-markierten Anti-Maus-IgG-Antikörper (Sigma) enthält. Anschließend wird wie nach der Anti-His-Tag-Inkubation gewaschen. In einem dritten Inkubationsschritt werden dann die gebundenen Antikörper mit einer NBT/BCIP-"Substrat-Lösung" (4 Nitrobluetetrazoliumchlorid / Chlorobromoindolylphosphate, EUROIMMUN) nachgewiesen.

### Ergebnisse:

Im Westernblot nach reduzierend durchgeführter SDS-PAGE kann die Expression der rekombinanten Konstrukte durch die Präsenz His-Tag-reaktiver Proteine nachgewiesen werden, deren Größen gut mit der auf Basis der Aminosäuresequenzen vorhergesagten Massen von 23-25 kDa übereinstimmen. Zellen, die den unveränderten Plasmidvektor enthalten, weisen kein entsprechendes Protein auf.

### Beispiel 2: Aufreinigung rekombinanter OspC-Varianten durch Affinitätschromatographie

Bei der Affinitätssäule handelt es sich um eine NiNTA-Spinsäule (Qiagen), die nach Empfehlungen des Herstellers gehandhabt wird.

Die gemäß Beispiel 1 geernteten Zellen werden erneut wie beschrieben zentrifugiert, in 1 ml "TNI10-Puffer" (5 mM Tris-HCl pH 8,0; 300 mM NaCl; 10 mM Imidazol) resuspendiert und durch dreimalige, einminütige Ultraschallbehandlung (Branson Sonifier, Stufe 7, MicroTip) aufgeschlossen. 0,5 ml des Überstandes werden auf eine mit "TNI10-Puffer" äquilibrierte NiNTA-Spinsäule aufgebracht.

Nicht- oder schwachbindende Proteine werden durch mehrmaliges Waschen mit 0,5 ml "TNI10-Puffer" von der Säule entfernt. Die Elution erfolgt mit 0,2 ml "TNI150-Puffer" (5 mM Tris-HCl pH 8,0; 300 mM NaCl; 150 mM Imidazol). Die Elutionsfraktion wird durch Westernblot wie in Beispiel 1 analysiert.

### Ergebnisse:

Die Eluate enthalten im Wesentlichen Proteine, die nach Färbung der Westernblotmembran mit Ponceau-S-Färbelösung jeweils Banden entsprechend einer Größe von etwa 23-25 kDa entsprechen. Nach Westernblot und Detektion des His-Tags ist eine Bande entsprechend derselben Größe zu sehen. Dieses Ergebnis deutet darauf hin, dass das exprimierte Protein nach der Chromatographie im Wesentlichen frei von *E. coli*-Bestandteilen vorliegt.

### Beispiel 3: Linienblots zur Bestimmung von anti-OspC-Antikörpern mit Hilfe der rekombinanten OspC-Varianten

Nitrocellulosemembranen werden mit einem in 10% (w/v) Glycerin verdünnten aufgereinigten rekombinanten OspC gemäß Beispiel 2 (Konzentration: 20 - 100 µg/ml) linienförmig beschichtet. Anschließend werden die Membranen über Nacht getrocknet, 1 Stunde mit "Universalpuffer" (EUROIMMUN) blockiert, auf einer Festphase fixiert, mit einem Skalpell im 90°-Winkel zu den OspC-Linien in etwa 2 mm breite Streifen geschnitten und anschließend in Aluminiumbeuteln mit Trockenbeuteln bis zur Benutzung bei 4 °C gelagert. Die Inkubation der Streifen erfolgt wie in der Arbeitsanleitung für EUROLINE-WB-Streifen (EUROIMMUN) angegeben, die als Vergleich inkubiert werden. Als weiterer Vergleich dient die Inkubation von recomLine Borrelia-Streifen (Mikrogen) gemäß Herstellerangaben.

Zur Ermittlung von Sensitivität und Spezifität werden 25 Serumproben von Patienten mit gesicherter früher Phase einer Borrelieninfektion sowie 50 Serumproben von Schwangeren (welche häufig unspezifische Antikörper aufweisen) ohne Anzeichen einer Borrelieninfektion und 25 Serumprobenpaare von gesunden Personen, die im Abstand von 14 Tagen entnommen wurden, hinsichtlich des Vorhandenseins OspC-spezifischer Antikörper der Immunglobulinklasse M (IgM) analysiert. Positive Reaktionen bei den gesunden Personen werden durch Analyse der IgG-Serokonversion (Vergleich Tag 0/Tag 14) mit mehreren Testsystemen zum Nachweis von Antikörpern der Klasse IgG gegen Borrelien, z.B. EUROLINE-WB IgG und Anti-Borrelia-ELISA IgG (EUROIMMUN), überprüft. Bei keiner positiven Reaktion erbrachte die Folgeprobe Hinweise auf eine Serokonversion.

**Tabelle 1:**

| | n | EUROLINE-WB IgM | His-Cys-m-OspC_{V5461} SEQ ID NO:2 | His-m-OSpC_{VS461} SEQ ID NO:1 | recomline Borrelia IgM _{Borrelia afzelii} |
|---|---|---|---|---|---|
| | | Anti-OspC IgM | | | |
| Frühe Phase [n] | 20 | 17 | 17 | 17 | 19 |
| Sensitivität[%] | 20 | 85 | 85 | 85 | 95 |
| gesunde Schwangere [n] | 50 | 2 | 2 | 6 | 8 |
| gesunde Blutspender [n] | 25 | 4 | 4 | 7 | 9 |
| Spezifität [%] | 75 | 92 | 92 | 82,7 | 77,3 |
| Diagnostische Genauigkeit [%] | 100 | 90,5 | 90,5 | 83,2 | 81,1 |

### Ergebnisse:

Die Ergebnisse sind beispielhaft für OspC aus *B. afzelii* in Tabelle 1 zusammengestellt.

Ein großer Anteil der Seren von Patienten in einer frühen Phase der Borrelieninfektion weisen IgM-Antikörper gegen OspC auf. In der Studie weisen der Westernblot mit dem aus *B. afzelii* stammenden nativen OspC und der Linienblot mit His-Cys-m-OSpC_{VS461} nahezu gleichwertige Sensitivitäten und Spezifitäten und gleichzeitig die höchsten diagnostischen Genauigkeiten auf. Der Linienblot mit der Variante His-m-OspC_{VS461} und das rekombinante OspC aus *B. afzelii* auf dem recomBlot Borrelia IgM zeichnen sich demgegenüber vor allem durch unspezifische Reaktionen bei den gesunden Personen aus.

### Beispiel 4: Analyse der rekombinanten OspC-Varianten

Gemäß Beispiel 2 werden die verschiedenen aufgereinigten OspC-Varianten durch SDS-PAGE aufgetrennt. Dabei erfolgt die Auftrennung sowohl unter nicht reduzierenden Bedingungen, unter reduzierenden Bedingungen in Anwesenheit von Dithiotreitol (DTT) und unter alkylierenden Bedingungen in Anwesenheit von Iodacetamid nach DTT-Reduktion. Im Anschluss wird ein Westernblot gemäß Beispiel 1 durchgeführt. Parallel zu dem in Beispiel 1 aufgeführten monoklonalen Antikörper gegen den His-Tag werden auch gemäß Beispiel 3 Anti-OspC-IgM positive Seren in einer 1:200 Verdünnung inkubiert. Im Fall humaner Seren wird im zweiten Inkubationsschritt ein 1:10 in "Universalpuffer" verdünntes Konjugat aus Anti-human-IgM und alkalischer Phosphatase (EUROIMMUN) verwendet.

### Ergebnisse:

Die Auftrennung unter reduzierenden und alkylierenden Bedingungen führt sowohl nach Ponceau-S-Färbung als auch nach Detektion des His-Tags gemäß Beispiel 1 jeweils zu Banden entsprechend einer Größe von etwa 23-25 kDa. Dies trifft auch für die Varianten His-m-OspC_{VS461}(SEQ ID NO: 1), His-m-OspC₂₀₀₄₇ (SEQ ID NO: 4) und His-m-OspC_{B31} (SEQ ID NO: 7) unter nicht reduzierenden Bedingungen zu. Demgegenüber sind bei den Varianten His-Cys-m-OspC_{VS461} (SEQ ID NO: 2), His-Cys-m-OspC₂₀₀₄₇ (SEQ ID NO: 5) und His-Cys-m-OspC_{B31} (SEQ ID NO: 8) jeweils zwei Banden bei 23-25 und 45-50 kDa vorhanden, die ungefähr jeweils gleich intensiv gefärbt sind. Diese Ergebnisse deuten darauf hin, dass die Varianten His-Cys-m-OspC_{VS461}, His-Cys-m-OspC₂₀₀₄₇ und His-Cys-m-OspC_{B31} jeweils als Dimere vorliegen, in denen jeweils zwei Monomere über Disulfidbrücken miteinander verknüft sind (s. Figur 2).

Nach Inkubation mit humanen Seren ist die Verteilung der Banden bei den jeweiligen Varianten identisch. Auffällig ist aber, dass die Banden bei 45-50 kDa jeweils wesentlich intensiver gefärbt sind, als die Banden bei 23-25 kDa. Die unterschiedlichen Färbeintensitäten nach Direktanfärbung durch Ponceau-S und immunologischer Färbung nach Inkubation mit humanen Seren deuten darauf hin, dass die Dimere jeweils höhere spezifische Reaktivitäten aufweisen als die Monomere (s. Figur 2).

### Beispiel 5: Herstellung von Heterodimeren

Aliquote von zwei der gemäß Beispiel 2 aufgereinigten OspC-Dimer-Varianten, z.B. His-m-Cys-OsPC_{VS461} und His-m-Cys-OspC₂₀₀₄₇, werden in gleichen Anteilen miteinander vermischt. Ein Aliquot des Gemisches wird zur Analytik aufbewahrt. Der Rest wird durch Zugabe von DTT reduziert und der Erfolg der Reduktion durch nicht reduzierende SDS-PAGE und Westernblot gemäß Beispiel 4 überprüft. Anschließend wird das Gemisch durch erschöpfende Dialyse gegen 20 mM Tris-HCl pH 8,5 von DTT befreit und für 24 Stunden durch eine Pipettenspitze mit Druckluft durchströmt, um ein oxidatives Milieu zu schaffen. Das Gemisch wird schließlich durch nicht reduzierende isoelektrische Fokussierung zwischen pH 6 und 9 mit dem ZoomRunner-System (Invitrogen) gemäß Herstellerangaben, anschließende reduzierende SDS-PAGE und Westernblot analysiert. Als Vergleich werden die beiden einzelnen OspC-Dimere und das Gemisch vor DTT-Zugabe analysiert.

### Ergebnisse:

His-m-Cys-OspC_{VS461} weist einen Spot entsprechend ca. pH 7,5 und 24 kDa, His-m-OspC₂₀₀₄₇ dagegen entsprechend ca. pH 6,5 und 24 kDa auf. Das Gemisch aus His-m-OspC_{VS461} und His-m-OspC₂₀₀₄₇ vor Reduktion weist zwei Spots entsprechend ca. pH 7,5 / 24 kDa und pH 6,5 / 24 kDa auf. Das reduzierte und anschließend Luftoxidierte Gemisch weist demgegenüber drei Spots entsprechend ca. pH 7,5 / 24 kDa, pH 6,8 / 24 kDa und pH 6,5 / 24 kDa auf. Die Ergebnisse deuten darauf hin, dass, zumindest teilweise, Heterodimere vorliegen.

### Beispiel 6: Immunogenität von Disulfid-verbrückten OspC-Dimeren

Die gemäß Beispiel 2 aufgereinigten Proteine His-m-OspC_{VS461} und His-Cys-m-OspC_{VS461} werden je 10 weiblichen C3H/He Mäusen im Alter von vier bis sechs Wochen erstmalig und dann im Abstand von 14, 28, und 42 Tagen in Portionen von je 100 µg pro Dosis subkutan ohne Adjuvans injiziert. Als Kontrolle werden weiteren 10 Mäusen jeweils 0,9% (w/v) Kochsalzlösung injiziert. Allen Tieren wird am Tag der ersten Injektion und dann nach 24, 38, 52 und 66 Tagen Blut abgenommen. OspC-spezifische Antikörper werden in einer 1:1000 Verdünnung des jeweiligen Serums mit Hilfe eines Anti-Borrelia IgM ELISA (EUROIMMUN) bestimmt, bei dem das Konjugat zum Nachweis gebundener humaner Antikörper gegen ein 1:2000 in Konjugatverdünnungspuffer (EUROIMMUN) verdünntes Konjugat zum Nachweis gebundener muriner Antikörper der Klasse IgG (Dianova) ausgetauscht wird. Von den Ergebnissen jeweils 10 gleichartig behandelter Tiere werden Mittelwert (MW) und Standardabweichung (σ) ermittelt.

Zur Überprüfung der Spezifität der Immunantwort werden die 30 Serumproben vom Tag der letzen Blutabnahme auf EUROLINE-WB-Streifen (EUROIMMUN) in einer 1:500 Verdünnung inkubiert und die gebundenen murinen Antikörper gemäß Versuch 1 nachgewiesen.

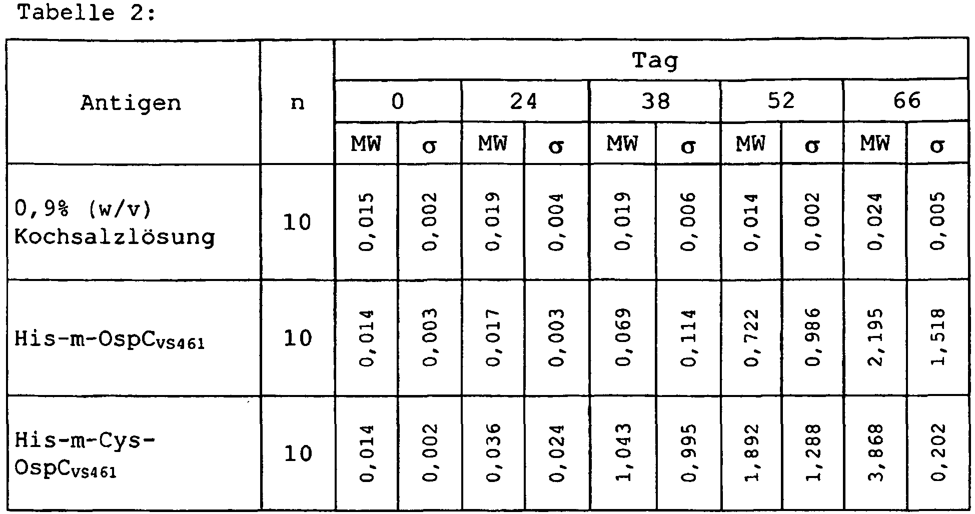

### Ergebnisse:

Die Ergebnisse werden in Extinktion bei 450 nm angegeben und sind in Tabelle 2 zusammengestellt.

Es zeigt sich, dass wesentlich schneller und höhere Messwerte erzeugt werden, wenn die Tiere mit der Variante His-m-Cys-OSpC_{VS461} immunisiert wurden. Des Weitern zeigt sich, dass am Ende des Immunisierungsprogramm alle Seren von Tieren, die mit der Variante His-m-Cys-OspC_{VS461} immunisiert wurden, hohe Messwerte nahe dem Mittelwert erzeugen. Demgegenüber deuten sehr niedrige oder niedrige Messwerte bei einigen Tieren, die mit der Variante His-m-OspC_{VS461} immunisiert wurden, daraufhin, dass die Immunisierung bei diesen Tieren nicht zu einem wesentlichen Antikörpertiter geführt hat.

Die Inkubation auf EUROLINE-WB-Streifen zeigt, dass alle Seren, die im ELISA eine erhöhte Extinktion (E₄₅₀ nm ≥ 0,3) erzeugen, eine Bande am Migrationsort des OspC erzeugen. Dabei korreliert die Bandenstärke in etwa mit der Extinktion.

Beide Ergebnisse zusammengenommen legen nahe, dass die Mäuse, die mit der Variante His-m-Cys-OspC_{VS461} immunisiert wurden, schneller Antikörper erzeugen als nach Immunisierung mit der Variante His-m-OspC_{VS461} und dass höhere Antikörperkonzentrationen am Ende der Immunisierung erreicht werden.

### Literaturliste

1 Cutler SJ, Wright DJ: Comparison of immunofluorescence and enzyme linked immunosorbent assays for diagnosing Lyme disease. J Clin Pathol 42:869-871 (1989).
2 Earnhart CG, Buckles EL, Dumler JS, Marconi RT: Demonstration of OspC type diversity in invasive human lyme disease isolates and identification of previously uncharacterized epitopes that define the specificity of the OspC murine antibody response. Infect Immun 73:7869-7877 (2005).
3 Eicken C, Sharma V, Klabunde T, Owens RT, Pikas DS, Hook M, Sacchettini JC: Crystal structure of Lyme disease antigen outer surface protein C from Borrelia burgdorferi. J Biol Chem 276:10010-10015 (2001).
4 Fister RD, Weymouth LA, McLaughlin JC, Ryan RW, Tilton RC: Comparative evaluation of three products for the detection of Borrelia burgdorferi antibody in human serum. J Clin Microbiol 27:2834-2837 (1989).
5 Fuchs R, Jauris S, Lottspeich F, Preac-Mursic V, Wilske B, Soutschek E: Molecular analysis and expression of a Borrelia burgdorferi gene encoding a 22 kDa protein (pC) in Escherichia coli. Mol Microbiol 6:503-509 (1992).
6 Hagman KE, Lahdenne P, Popova TG, Porcella SF, Akins DR, Radolf JD, Norgard MV: Decorin-binding protein of Borrelia burgdorferi is encoded within a two-gene operon and is protective in the murine model of Lyme borreliosis. Infect Immun 66:2674-2683 (1998).
7 Hansen K, Hindersson P, Pedersen NS: Measurement of antibodies to the Borrelia burgdorferi flagellum improves serodiagnosis in Lyme disease. J Clin Microbiol 26:338-346 (1988).
8 Hauser U, Lehnert G, Wilske B: Diagnostic value of proteins of three Borrelia species (Borrelia burgdorferi sensu lato) and implications for development and use of recombinant antigens for serodiagnosis of Lyme borreliosis in Europe. Clin Diagn Lab Immunol 5:456-462 (1998).
9 Hyde JA, Trzeciakowski JP, Skare JT: Borrelia burgdorferi alters its gene expression and antigenic profile in response to CO2 levels. J Bacteriol 189:437-445 (2007).
10 Kumaran D, Eswaramoorthy S, Luft BJ, Koide S, Dunn JJ, Lawson CL, Swaminathan S: Crystal structure of outer surface protein C (OspC) from the Lyme disease spirochete, Borrelia burgdorferi. EMBO J 20:971-978 (2001).
11 Lawrenz MB, Hardham JM, Owens RT, Nowakowski J, Steere AC, Wormser GP, Norris SJ: Human antibody responses to VlsE antigenic variation protein of Borrelia burgdorferi. J Clin Microbiol 37:3997-4004 (1999).
12 Mathiesen MJ, Christiansen M, Hansen K, Holm A, Asbrink E, Theisen M: Peptide-based OspC enzyme-linked immunosorbent assay for serodiagnosis of Lyme borreliosis. J Clin Microbiol 36:3474-3479 (1998a).
13 Mathiesen MJ, Holm A, Christiansen M, Blom J, Hansen K, Ostergaard S, Theisen M: The dominant epitope of Borrelia garinii outer surface protein C recognized by sera from patients with neuroborreliosis has a surface-exposed conserved structural motif. Infect Immun 66:4073-4079 (1998b).
14 Norris SJ, Carter CJ, Howell JK, Barbour AG: Low-passageassociated proteins of Borrelia burgdorferi B31: characterization and molecular cloning of OspD, a surface-exposed, plasmid-encoded lipoprotein. Infect Immun 60:4662-4672 (1992).
15 Pollack RJ, Telford SR, III, Spielman A: Standardization of medium for culturing Lyme disease spirochetes. J Clin Microbiol 31:1251-1255 (1993).
16 Ruzic-Sabljic E, Strle F: Comparison of growth of Borrelia afzelii, B. garinii, and B. burgdorferi sensu stricto in MKP and BSK-II medium. Int J Med Microbiol 294:407-412 (2004).
17 Seshu J, Boylan JA, Gherardini FC, Skare JT: Dissolved oxygen levels alter gene expression and antigen profiles in Borrelia burgdorferi. Infect Immun 72:1580-1586 (2004).
18 Wilske B, Fingerle V: Lyme-Borreliose Diagnostik 2005).
19 Wilske B, Jauris-Heipke S, Lobentanzer R, Pradel I, Preac-Mursic V, Rossler D, Soutschek E, Johnson RC: Phenotypic analysis of outer surface protein C (OspC) of Borrelia burgdorferi sensu lato by monoclonal antibodies: relationship to genospecies and OspA serotype. J Clin Microbiol 33:103-109 (1995).
20 Wilske B, Preac-Mursic V, Jauris S, Hofmann A, Pradel I, Soutschek E, Schwab E, Will G, Wanner G: Immunological and molecular polymorphisms of OspC, an immunodominant major outer surface protein of Borrelia burgdorferi. Infect Immun 61:2182-2191 (1993).
21 Wu HC, Tokunaga M: Biogenesis of lipoproteins in bacteria. Curr Top Microbiol Immunol 125:127-157 (1986).
22 Yang X, Popova TG, Goldberg MS, Norgard MV: Influence of cultivation media on genetic regulatory patterns in Borrelia burgdorferi. Infect Immun 69:4159-4163 (2001).
23 WO 9742221
24 EP1741718

## Patentansprüche

1. Protein, umfassend ein erstes OspC-Polypeptid, **dadurch gekennzeichnet, dass** das erste OspC-Polypeptid über eine Disulfid-Brücke mit einem zweiten OspC-Polypeptid verbunden ist, wobei die Disulfidbrücke durch Verbindung von einem Cystein entsteht, das nicht mehr als 100 Aminosäurepositionen vom N-Terminus des OspC-Polypeptids entfernt ist,
wobei das erste und das zweite OspC-Polypeptid eine Aminosäureidentität von mindestens 70% mit SEQ ID NO: 3, SEQ ID NO: 6 oder SEQ ID NO: 9 aufweisen.

2. Protein nach Anspruch 1, **dadurch gekennzeichnet, dass** das erste und/oder das zweite OspC-Polypeptid eine Aminosäureidentität von mindestens 90% mit SEQ ID NO: 3, SEQ ID NO: 6 oder SEQ ID NO: 9 aufweist und spezifisch von Antikörper gegen OspC aus Borrelien erkannt wird.

3. Protein nach Anspruch 1 oder 2, **dadurch gekennzeichnet, dass** das erste und/oder das zweite OspC-Polypeptid mindestens ein Epitop umfasst, welches spezifisch von Antikörper gegen OspC aus Borrelien erkannt wird, wobei das Epitop mindestens eine Teilsequenz von 10 fortlaufenden Aminosäuren aus SEQ ID NO: 3, SEQ ID NO: 6 oder SEQ ID NO: 9 umfasst.

4. Protein nach einem der Ansprüche 1 bis 3, **dadurch gekennzeichnet, dass** das erste und/oder das zweite OspC-Polypeptid C-terminal eine der Aminosäuresequenzen gemäß SEQ ID NO: 3, SEQ ID NO: 6 oder SEQ ID NO: 9 aufweist.

5. Protein nach einem der Ansprüche 1 bis 4, **dadurch gekennzeichnet, dass** das erste und/oder das zweite OspC-Polypeptid keine Erkennungssequenz für eine Acylierung aufweist.

6. Protein nach einem der Ansprüche 1 bis 5, **dadurch gekennzeichnet, dass** die Disulfidbrücke durch Verbindung von einem Cystein entsteht, das nicht mehr als 30 Aminosäurepositionen vom N-Terminus des ersten und/oder des zweiten OspC-Polypeptids entfernt ist.

7. Protein nach einem der Ansprüche 1 bis 6, **dadurch gekennzeichnet, dass** das erste und/oder das zweite OspC- Polypeptid eine Sequenz nach SEQ ID NO: 2, SEQ ID NO: 5 oder SEQ ID NO: 8 aufweist.

8. Protein nach einem der Ansprüche 1 bis 7, **dadurch gekennzeichnet, dass** durch die Disulfidbrücke ein Homodimer von zwei gleichen OspC-Polypeptiden oder ein Heterodimer unterschiedlicher OspC-Polypeptide entsteht.

9. Protein nach einem der Ansprüche 1 bis 8, **dadurch gekennzeichnet, dass** sich die Erkennung des Polypeptids durch Antikörper gegen OspC aus Borrelien durch in Kontakt bringen mit mindestens einem Thiol-haltigen Reagenz, gegebenenfalls in Kombination mit einem Alkylhalogenid, verringert.

10. Verfahren zum Nachweis von Antikörpern gegen ein Protein nach einem der Ansprüche 1 bis 9, bei dem man eine biologische Probe mit dem Protein nach einem der Ansprüche 1 bis 9 in Kontakt bringt und die Bindung von Antikörpern an das Protein nachweist.

11. Verfahren zur Diagnose einer Borrelieninfektion, **dadurch gekennzeichnet, dass** man eine biologische Probe eines Patienten mit einem Protein nach einem der Ansprüche 1 bis 9 in Kontakt bringt und die Bindung von Antikörpern an das Protein nachweist, wobei ein Nachweis der Bindung von Antikörpern auf eine Borrelieninfektion hinweist.

12. Verfahren nach einem der Ansprüche 10 oder 11, **dadurch gekennzeichnet, dass** man die Bindung der Antikörper mit einem Immunfluoreszenztest, ELISA, Lumineszenztest, Westernblot, Linienblot oder Dot Blot nachweist.

13. Kit zur Diagnose einer Borrelieninfektion, umfassend ein Protein nach einem der Ansprüche 1 bis 9.

14. Verwendung eines Proteins nach einem der Ansprüche 1 bis 9 zur Herstellung eines Impfstoffs gegen eine Borrelieninfektion.

15. Impfstoff gegen eine Borrelieninfektion, umfassend ein Protein nach einem der Ansprüche 1 bis 9.

## Claims

1. A protein, comprising a first OspC polypeptide, **characterized in that** said first OspC polypeptide is linked with a second OspC polypeptide via a disulfide bridge, wherein said disulfide bridge is formed by binding of a cysteine, which is no more than 100 amino acid positions away from the N-terminus of the OspC polypeptide,
wherein said first and second OspC polypeptides have an amino acid identity of at least 70 % with SEQ ID NO: 3, SEQ ID NO: 6, or SEQ ID NO: 9.

2. The protein according to claim 1, **characterized in that** said first and/or second OspC polypeptide has an amino acid identity of at least 90 % with SEQ ID NO: 3, SEQ ID NO: 6, or SEQ ID NO: 9 and is specifically detected by antibodies against OspC from borrelia.

3. The protein according to claims 1 or 2, **characterized in that** said first and/or second OspC polypeptide comprises at least one epitope, which is specifically detected by antibodies against OspC from borrelia, wherein said epitope comprises at least a partial sequence of 10 consecutive amino acids from SEQ ID NO: 3, SEQ ID NO: 6, or SEQ ID NO: 9.

4. The protein according to any of claims 1 to 3, **characterized in that** said first and/or second OspC polypeptide has an amino acid sequence according to SEQ ID NO: 3, SEQ ID NO: 6, or SEQ ID NO: 9 at its C-terminus.

5. The protein according to any of claims 1 to 4, **characterized in that** said first and/or second OspC polypeptide has no signal sequence for an acylation.

6. The protein according to any of claims 1 to 5, **characterized in that** said disulfide bridge is formed by binding of a cysteine, which is no more than 30 amino acid positions away from the N-terminus of said first and/or second OspC polypeptide.

7. The protein according to any of claims 1 to 6, **characterized in that** said first and/or second OspC polypeptide has a sequence according to SEQ ID NO: 2, SEQ ID NO: 5, or SEQ ID NO: 8.

8. The protein according to any of claims 1 to 7, **characterized in that** by means of said disulfide bridge, a homodimer of two equal OspC polypeptides or a heterodimer of different OspC polypeptides is formed.

9. The protein according to any of claims 1 to 8, **characterized in that** said detection of said polypeptide by antibodies against OspC from borrelia is reduced by contacting with at least one thiol-containing reagent, if necessary in combination with an alkyl halide.

10. A method for detecting antibodies against a protein according to any of claims 1 to 9, wherein a biological sample is contacted with said protein according to any of claims 1 to 9, and the binding of antibodies to said protein is detected.

11. A method for diagnosing a borrelia infection, **characterized in that** a biological sample of a patient is contacted with a protein according to any of claims 1 to 9, and said binding of antibodies to said protein is detected, wherein detection of said binding of antibodies indicates a borrelia infection.

12. The method according to any of claims 10 or 11, **characterized in that** said binding of said antibodies is detected with an immunofluorescence test, ELISA, luminescence test, Western blot, line blot, or dot blot.

13. A kit for diagnosing a borrelia infection, comprising a protein according to any of claims 1 to 9.

14. Use of a protein according to any of claims 1 to 9 for manufacturing a vaccine against a borrelia infection.

15. A vaccine against a borrelia infection, comprising a protein according to any of claims 1 to 9.

## Revendications

1. Protéine, comportant un premier polypeptide OspC, caractérisée en ceci que ledit premier polypeptide OspC est relié par l'intermédiaire d'un pont disulfure à un deuxième polypeptide OspC, ledit pont disulfure étant créé par la composition d'une cystéine qui n'est pas éloignée de plus de 100 positions aminées de l'extrémité N-terminale dudit polypeptide OspC,
ledit premier et ledit deuxième polypeptide OspC présentant une identité d'acide aminé d'au moins 70%, avec SEQ ID n° : 3, SEQ ID n° : 6 ou SEQ ID n° : 9.

2. Protéine selon la revendication 1, caractérisée en ceci que ledit premier et/ou ledit deuxième polypeptide OspC présente une identité d'acide aminé d'au moins 90%, avec SEQ ID n° : 3, SEQ ID n° : 6 ou SEQ ID n° : 9 et est spécifiquement détecté par les anticorps dirigés contre les OspC provenant de Borrelia.

3. Protéine selon la revendication 1 ou 2, caractérisée en ceci que ledit premier et/ou ledit deuxième polypeptide OspC comporte au moins un épitope qui est spécifiquement détecté par les anticorps dirigés contre les OspC provenant de Borrelia, ledit épitope comportant au moins une séquence partielle composée de 10 acides aminés continus de SEQ ID n° : 3, SEQ ID n° : 6 ou SEQ ID n° : 9.

4. Protéine selon l'une des revendications 1 à 3, caractérisée en ceci que ledit premier et/ou ledit deuxième polypeptide OspC présente, à son extrémité C-terminale, l'une des séquences d'acide aminé selon SEQ ID n° : 3, SEQ ID n° : 6 ou SEQ ID n° : 9.

5. Protéine selon l'une des revendications 1 à 4, caractérisée en ceci que ledit premier et/ou le deuxième polypeptide OspC ne présente pas de séquence de détection d'une acylation.

6. Protéine selon l'une des revendications 1 à 5, caractérisée en ceci que ledit pont disulfure est créé par la composition d'une cystéine qui n'est pas éloignée de plus de 30 positions aminées de l'extrémité N-terminale dudit premier et/ou dudit deuxième polypeptide OspC.

7. Protéine selon l'une des revendications 1 à 6, caractérisée en ceci que ledit premier et/ou ledit deuxième polypeptide OspC présente une séquence selon SEQ ID n° : 2, SEQ ID n° : 5 ou SEQ ID n° : 8.

8. Protéine selon l'une des revendications 1 à 7, caractérisée en ceci qu'au moyen dudit pont disulfure un homodimère composé de deux polypeptides OspC identiques ou un hétérodimère composé de polypeptides OspC différents est formé.

9. Protéine selon l'une des revendications 1 à 8, caractérisée en ceci que la détection dudit polypeptide par les anticorps dirigés contre les OspC provenant de Borrelia est diminuée par une mise en contact avec au moins un réactif contenant du thioalcool, si nécessaire, en combinaison avec un halogénure d'alkyle.

10. Procédé destiné à détecter des anticorps dirigés contre une protéine selon l'une des revendications 1 à 9 dans le cadre duquel un échantillon biologique est mis en contact avec une protéine selon l'une des revendications 1 à 9 et la liaison des anticorps à ladite protéine est détectée.

11. Procédé destiné à diagnostiquer une infection type Borrelia, caractérisé en ceci qu'un échantillon biologique d'un patient est mis en contact avec une protéine selon l'une des revendications 1 à 9 et que la liaison des anticorps à ladite protéine est détectée, ladite détection de ladite liaison des anticorps indiquant une infection type Borrelia.

12. Procédé selon l'une des revendications 10 ou 11, caractérisé en ceci que ladite liaison des anticorps est détectée au moyen d'un test d'immunofluorescence, de l'ELISA, d'un test de luminescence, d'un western blot, d'un line blot ou d'un dot blot.

13. Kit destiné à diagnostiquer une infection type Borrelia comportant une protéine selon l'une des revendications 1 à 9.

14. Utilisation d'une protéine selon l'une des revendications 1 à 9 pour la préparation d'un vaccin contre une infection type Borrelia.

15. Vaccin contre une infection type Borrelia, comportant une protéine selon l'une des revendications 1 à 9.


### SEQUENCE LISTING

<110> EUROIMMUN AG
   Komorowski, Lars
   Janssen, Anthonina
   Probst, Christian
<120> Polypeptide und Verfahren zur spezifischen Detektion von Antikörpern bei Patienten mit einer Borrelieninfektion
<130> ?
<160> 24
<170> PatentIn version 3.3
<210> 1
   <211> 203
   <212> PRT
   <213> Artificial
<220>
   <223> Generated by genetic engineering.
<400> 1
   Met Ser His His His His His His His His Ser Met Gly Asn Asn Ser
   1               5                   10                  15
   Gly Lys Gly Gly Asp Ile Ala Ser Thr Asn Pro Asp Glu Ser Ala Lys
               20                  25                  30
   Gly Pro Asn Leu Thr Glu Ile Ser Lys Lys Ile Thr Asp Ser Asn Ala
           35                  40                  45
   Val Val Leu Ala Val Lys Glu Val Glu Ala Leu Leu Ser Ser Ile Asp
       50                  55                  60
   Glu Leu Ala Lys Thr Ile Gly Lys Lys Ile Glu Ala Asn Gly Leu Gly
   65                  70                  75                  80
   Asn Glu Ala Asp Lys Asn Gly Ser Leu Leu Ala Gly Ala Tyr Ala Ile
                   85                  90                  95
   Ser Thr Leu Ile Lys Gln Lys Leu Asp Gly Leu Lys Gly Leu Glu Gly
               100                 105                 110
   Leu Asn Lys Glu Ile Ala Glu Ala Lys Lys Cys Ser Glu Ala Phe Thr
           115                 120                 125
   Lys Lys Leu Gln Asp Ser Asn Ala Asp Leu Gly Lys His Asn Ala Thr
       130                 135                 140
   Asp Ala Asp Ser Lys Glu Ala Ile Leu Lys Thr Asn Gly Thr Lys Thr
   145                 150                 155                 160
   Lys Gly Ala Lys Glu Leu Glu Glu Leu Phe Lys Ser Val Glu Ser Leu
                   165                 170                 175
   Ser Lys Ala Ala Lys Glu Ala Leu Ser Asn Ser Val Lys Glu Leu Thr
               180                 185                 190
   Ser Pro Val Val Ala Glu Ser Pro Lys Lys Pro
           195                 200
<210> 2
   <211> 203
   <212> PRT
   <213> Artificial
<220>
   <223> Generated by genetic engineering.
<400> 2
   Met Ser His His His His His His His His Ser Met Cys Asn Asn Ser
   1               5                   10                  15
   Gly Lys Gly Gly Asp Ile Ala Ser Thr Asn Pro Asp Glu Ser Ala Lys
               20                  25                  30
   Gly Pro Asn Leu Thr Glu Ile Ser Lys Lys Ile Thr Asp Ser Asn Ala
           35                  40                  45
   Val Val Leu Ala Val Lys Glu Val Glu Ala Leu Leu Ser Ser Ile Asp
       50                  55                  60
   Glu Leu Ala Lys Thr Ile Gly Lys Lys Ile Glu Ala Asn Gly Leu Gly
   65                  70                  75                  80
   Asn Glu Ala Asp Lys Asn Gly Ser Leu Leu Ala Gly Ala Tyr Ala Ile
                   85                  90                  95
   Ser Thr Leu Ile Lys Gln Lys Leu Asp Gly Leu Lys Gly Leu Glu Gly
               100                 105                 110
   Leu Asn Lys Glu Ile Ala Glu Ala Lys Lys Cys Ser Glu Ala Phe Thr
           115                 120                 125
   Lys Lys Leu Gln Asp Ser Asn Ala Asp Leu Gly Lys His Asn Ala Thr
       130                 135                 140
   Asp Ala Asp Ser Lys Glu Ala Ile Leu Lys Thr Asn Gly Thr Lys Thr
   145                 150                 155                 160
   Lys Gly Ala Lys Glu Leu Glu Glu Leu Phe Lys Ser Val Glu Ser Leu
                   165                 170                 175
   Ser Lys Ala Ala Lys Glu Ala Leu Ser Asn Ser Val Lys Glu Leu Thr
               180                 185                 190
   Ser Pro Val Val Ala Glu Ser Pro Lys Lys Pro
           195                 200
<210> 3
   <211> 191
   <212> PRT
   <213> Borrelia afzelii VS461
<400> 3
   Cys Asn Asn Ser Gly Lys Gly Gly Asp Ile Ala Ser Thr Asn Pro Asp
   1               5                   10                  15
   Glu Ser Ala Lys Gly Pro Asn Leu Thr Glu Ile Ser Lys Lys Ile Thr
               20                  25                  30
   Asp Ser Asn Ala Val Val Leu Ala Val Lys Glu Val Glu Ala Leu Leu
           35                  40                  45
   Ser Ser Ile Asp Glu Leu Ala Lys Thr Ile Gly Lys Lys Ile Glu Ala
       50                  55                  60
   Asn Gly Leu Gly Asn Glu Ala Asp Lys Asn Gly Ser Leu Leu Ala Gly
   65                  70                  75                  80
   Ala Tyr Ala Ile Ser Thr Leu Ile Lys Gln Lys Leu Asp Gly Leu Lys
                   85                  90                  95
   Gly Leu Glu Gly Leu Asn Lys Glu Ile Ala Glu Ala Lys Lys Cys Ser
               100                 105                 110
   Glu Ala Phe Thr Lys Lys Leu Gln Asp Ser Asn Ala Asp Leu Gly Lys
           115                 120                 125
   His Asn Ala Thr Asp Ala Asp Ser Lys Glu Ala Ile Leu Lys Thr Asn
       130                 135                 140
   Gly Thr Lys Thr Lys Gly Ala Lys Glu Leu Glu Glu Leu Phe Lys Ser
   145                 150                 155                 160
   Val Glu Ser Leu Ser Lys Ala Ala Lys Glu Ala Leu Ser Asn Ser Val
                   165                 170                 175
   Lys Glu Leu Thr Ser Pro Val Val Ala Glu Ser Pro Lys Lys Pro
               180                 185                 190
<210> 4
   <211> 203
   <212> PRT
   <213> Artificial
<220>
   <223> Generated by genetic engineering.
<400> 4
   Met Ser His His His His His His His His Ser Met Gly Asn Asn Ser
   1               5                   10                  15
   Gly Gly Asp Thr Ala Ser Thr Asn Pro Asp Glu Ser Val Lys Gly Pro
               20                  25                  30
   Asn Leu Thr Glu Ile Ser Lys Lys Ile Thr Asp Ser Asn Ala Phe Val
           35                  40                  45
   Leu Ala Val Lys Glu Val Glu Ala Leu Ile Ser Ser Ile Asp Glu Leu
       50                  55                  60
   Ala Lys Ala Ile Gly Gln Arg Ile Gln Gln Asn Gly Leu Val Ala Asp
   65                  70                  75                  80
   Ala Gly His Asn Ser Ala Leu Leu Ala Gly Ala His Glu Ile Ser Ile
                   85                  90                  95
   Leu Ile Thr Gln Lys Leu Asp Gly Leu Lys Gly Leu Glu Gly Leu Lys
               100                 105                 110
   Ala Glu Ile Ala Glu Ala Lys Lys Tyr Ser Glu Ala Phe Thr Lys Lys
           115                 120                 125
   Leu Lys Asp Asn His Ala Gln Leu Gly Ile Gln Asn Gly Ala Ser Leu
       130                 135                 140
   Asp Asp Glu Ala Lys Lys Ala Ile Leu Lys Thr Asn Val Asp Lys Thr
   145                 150                 155                 160
   Lys Gly Ala Glu Glu Leu Glu Lys Leu Phe Lys Ser Val Glu Ser Leu
                   165                 170                 175
   Ser Lys Ala Ala Gln Glu Ala Leu Thr Asn Ser Val Lys Glu Leu Thr
               180                 185                 190
   Asn Pro Val Val Ala Glu Thr Pro Lys Lys Pro
           195                 200
<210> 5
   <211> 203
   <212> PRT
   <213> Artificial
<220>
   <223> Generated by genetic engineering.
<400> 5
   Met Ser His His His His His His His His Ser Met Cys Asn Asn Ser
   1               5                   10                  15
   Gly Gly Asp Thr Ala Ser Thr Asn Pro Asp Glu Ser Val Lys Gly Pro
               20                  25                  30
   Asn Leu Thr Glu Ile Ser Lys Lys Ile Thr Asp Ser Asn Ala Phe Val
           35                  40                  45
   Leu Ala Val Lys Glu Val Glu Ala Leu Ile Ser Ser Ile Asp Glu Leu
       50                  55                  60
   Ala Lys Ala Ile Gly Gln Arg Ile Gln Gln Asn Gly Leu Val Ala Asp
   65                  70                  75                  80
   Ala Gly His Asn Ser Ala Leu Leu Ala Gly Ala His Glu Ile Ser Ile
                   85                  90                  95
   Leu Ile Thr Gln Lys Leu Asp Gly Leu Lys Gly Leu Glu Gly Leu Lys
               100                 105                 110
   Ala Glu Ile Ala Glu Ala Lys Lys Tyr Ser Glu Ala Phe Thr Lys Lys
           115                 120                 125
   Leu Lys Asp Asn His Ala Gln Leu Gly Ile Gln Asn Gly Ala Ser Leu
       130                 135                 140
   Asp Asp Glu Ala Lys Lys Ala Ile Leu Lys Thr Asn Val Asp Lys Thr
   145                 150                 155                 160
   Lys Gly Ala Glu Glu Leu Glu Lys Leu Phe Lys Ser Val Glu Ser Leu
                   165                 170                 175
   Ser Lys Ala Ala Gln Glu Ala Leu Thr Asn Ser Val Lys Glu Leu Thr
               180                 185                 190
   Asn Pro Val Val Ala Glu Thr Pro Lys Lys Pro
           195                 200
<210> 6
   <211> 191
   <212> PRT
   <213> Borrelia garinii 20047
<400> 6
   Cys Asn Asn Ser Gly Gly Asp Thr Ala Ser Thr Asn Pro Asp Glu Ser
   1               5                   10                  15
   Val Lys Gly Pro Asn Leu Thr Glu Ile Ser Lys Lys Ile Thr Asp Ser
               20                  25                  30
   Asn Ala Phe Val Leu Ala Val Lys Glu Val Glu Ala Leu Ile Ser Ser
           35                  40                  45
   Ile Asp Glu Leu Ala Lys Ala Ile Gly Gln Arg Ile Gln Gln Asn Gly
       50                  55                  60
   Leu Val Ala Asp Ala Gly His Asn Ser Ala Leu Leu Ala Gly Ala His
   65                  70                  75                  80
   Glu Ile Ser Ile Leu Ile Thr Gln Lys Leu Asp Gly Leu Lys Gly Leu
                   85                  90                  95
   Glu Gly Leu Lys Ala Glu Ile Ala Glu Ala Lys Lys Tyr Ser Glu Ala
               100                 105                 110
   Phe Thr Lys Lys Leu Lys Asp Asn His Ala Gln Leu Gly Ile Gln Asn
           115                 120                 125
   Gly Ala Ser Leu Asp Asp Glu Ala Lys Lys Ala Ile Leu Lys Thr Asn
       130                 135                 140
   Val Asp Lys Thr Lys Gly Ala Glu Glu Leu Glu Lys Leu Phe Lys Ser
   145                 150                 155                 160
   Val Glu Ser Leu Ser Lys Ala Ala Gln Glu Ala Leu Thr Asn Ser Val
                   165                 170                 175
   Lys Glu Leu Thr Asn Pro Val Val Ala Glu Thr Pro Lys Lys Pro
               180                 185                 190
<210> 7
   <211> 204
   <212> PRT
   <213> Artificial
<220>
   <223> Generated by genetic engineering.
<400> 7
   Met Ser His His His His His His His His Ser Met Gly Asn Asn Ser
   1               5                   10                  15
   Gly Lys Asp Gly Asn Thr Ser Ala Asn Ser Ala Asp Glu Ser Val Lys
               20                  25                  30
   Gly Pro Asn Leu Thr Glu Ile Ser Lys Lys Ile Thr Asp Ser Asn Ala
           35                  40                  45
   Val Leu Leu Ala Val Lys Glu Val Glu Ala Leu Leu Ser Ser Ile Asp
       50                  55                  60
   Glu Ile Ala Ala Lys Ala Ile Gly Lys Lys Ile His Gln Asn Asn Gly
   65                  70                  75                  80
   Leu Asp Thr Glu Asn Asn His Asn Gly Ser Leu Leu Ala Gly Ala Tyr
                   85                  90                  95
   Ala Ile Ser Thr Leu Ile Lys Gln Lys Leu Asp Gly Leu Lys Asn Glu
               100                 105                 110
   Gly Leu Lys Glu Lys Ile Asp Ala Ala Lys Lys Cys Ser Glu Thr Phe
           115                 120                 125
   Thr Asn Lys Leu Lys Glu Lys His Thr Asp Leu Gly Lys Glu Gly Val
       130                 135                 140
   Thr Asp Ala Asp Ala Lys Glu Ala Ile Leu Lys Thr Asn Gly Thr Lys
   145                 150                 155                 160
   Thr Lys Gly Ala Glu Glu Leu Gly Lys Leu Phe Glu Ser Val Glu Val
                   165                 170                 175
   Leu Ser Lys Ala Ala Lys Glu Met Leu Ala Asn Ser Val Lys Glu Leu
               180                 185                 190
   Thr Ser Pro Val Val Ala Glu Ser Pro Lys Lys Pro
           195                 200
<210> 8
   <211> 204
   <212> PRT
   <213> Artificial
<220>
   <223> Generated by genetic engineering.
<400> 8
   Met Ser His His His His His His His His Ser Met Cys Asn Asn Ser
   1 5 10 15
   Gly Lys Asp Gly Asn Thr Ser Ala Asn Ser Ala Asp Glu Ser Val Lys
   20 25 30
   Gly Pro Asn Leu Thr Glu Ile Ser Lys Lys Ile Thr Asp Ser Asn Ala
   35 40 45
   Val Leu Leu Ala Val Lys Glu Val Glu Ala Leu Leu Ser Ser Ile Asp
   50 55 60
   Glu Ile Ala Ala Lys Ala Ile Gly Lys Lys Ile His Gln Asn Asn Gly
   65 70 75 80
   Leu Asp Thr Glu Asn Asn His Asn Gly Ser Leu Leu Ala Gly Ala Tyr
   85 90 95
   Ala Ile Ser Thr Leu Ile Lys Gln Lys Leu Asp Gly Leu Lys Asn Glu
   100 105 110
   Gly Leu Lys Glu Lys Ile Asp Ala Ala Lys Lys Cys Ser Glu Thr Phe
   115 120 125
   Thr Asn Lys Leu Lys Glu Lys His Thr Asp Leu Gly Lys Glu Gly Val
   130 135 140
   Thr Asp Ala Asp Ala Lys Glu Ala Ile Leu Lys Thr Asn Gly Thr Lys
   145 150 155 160
   Thr Lys Gly Ala Glu Glu Leu Gly Lys Leu Phe Glu Ser Val Glu Val
   165 170 175
   Leu Ser Lys Ala Ala Lys Glu Met Leu Ala Asn Ser Val Lys Glu Leu
   180 185 190
   Thr Ser Pro Val Val Ala Glu Ser Pro Lys Lys Pro
   195 200
<210> 9
   <211> 192
   <212> PRT
   <213> Borrelia burgdorferi B31
<400> 9
   Cys Asn Asn Ser Gly Lys Asp Gly Asn Thr Ser Ala Asn Ser Ala Asp
   1 5 10 15
   Glu Ser Val Lys Gly Pro Asn Leu Thr Glu Ile Ser Lys Lys Ile Thr
   20 25 30
   Asp Ser Asn Ala Val Leu Leu Ala Val Lys Glu Val Glu Ala Leu Leu
   35 40 45
   Ser Ser Ile Asp Glu Ile Ala Ala Lys Ala Ile Gly Lys Lys Ile His
   50 55 60
   Gln Asn Asn Gly Leu Asp Thr Glu Asn Asn His Asn Gly Ser Leu Leu
   65 70 75 80
   Ala Gly Ala Tyr Ala Ile Ser Thr Leu Ile Lys Gln Lys Leu Asp Gly
   85 90 95
   Leu Lys Asn Glu Gly Leu Lys Glu Lys Ile Asp Ala Ala Lys Lys Cys
   100 105 110
   Ser Glu Thr Phe Thr Asn Lys Leu Lys Glu Lys His Thr Asp Leu Gly
   115 120 125
   Lys Glu Gly Val Thr Asp Ala Asp Ala Lys Glu Ala Ile Leu Lys Thr
   130 135 140
   Asn Gly Thr Lys Thr Lys Gly Ala Glu Glu Leu Gly Lys Leu Phe Glu
   145 150 155 160
   Ser Val Glu Val Leu Ser Lys Ala Ala Lys Glu Met Leu Ala Asn Ser
   165 170 175
   Val Lys Glu Leu Thr Ser Pro Val Val Ala Glu Ser Pro Lys Lys Pro
   180 185 190
<210> 10
   <211> 609
   <212> DNA
   <213> Synthetic
<400> 10
   atgagccatc atcatcatca tcatcatcat tccatgggta ataattcagg gaaaggtggg     60
   gatattgcat ctactaatcc tgatgagtct gcgaaaggac ctaatcttac agaaataagc    120
   aaaaaaatta cagattccaa tgcagttgta ctagctgtga aagaagttga ggctttgctt    180
   tcatctatag atgaacttgc taaaactatt ggtaaaaaaa tagaggcaaa tggtttgggt    240
   aacgaagcgg ataaaaacgg atcattatta gcaggagcct atgcaatatc aaccctaata    300
   aaacaaaaat tagatggatt gaaaggtcta gaaggattaa ataaagaaat tgcggaggcc    360
   aagaaatgtt ccgaagcatt tactaaaaag ctacaagata gtaacgcaga tcttggaaaa    420
   cataatgcta ctgatgctga ttcaaaagaa gcaattttga aaacaaatgg gactaaaact    480
   aagggtgcta aagaacttga agagttgttt aaatcagtag aaagcttgtc aaaagcagct    540
   aaagaagcat taagtaattc agttaaagag cttacaagcc ctgttgtggc agaaagtcca    600
   aaaaaacct                                                            609
<210> 11
   <211> 609
   <212> DNA
   <213> Synthetic
<400> 11
   atgagccatc atcatcatca tcatcatcat tccatgtgta ataattcagg gaaaggtggg     60
   gatattgcat ctactaatcc tgatgagtct gcgaaaggac ctaatcttac agaaataagc    120
   aaaaaaatta cagattccaa tgcagttgta ctagctgtga aagaagttga ggctttgctt    180
   tcatctatag atgaacttgc taaaactatt ggtaaaaaaa tagaggcaaa tggtttgggt    240
   aacgaagcgg ataaaaacgg atcattatta gcaggagcct atgcaatatc aaccctaata    300
   aaacaaaaat tagatggatt gaaaggtcta gaaggattaa ataaagaaat tgcggaggcc    360
   aagaaatgtt ccgaagcatt tactaaaaag ctacaagata gtaacgcaga tcttggaaaa    420
   cataatgcta ctgatgctga ttcaaaagaa gcaattttga aaacaaatgg gactaaaact    480
   aagggtgcta aagaacttga agagttgttt aaatcagtag aaagcttgtc aaaagcagct    540
   aaagaagcat taagtaattc agttaaagag cttacaagcc ctgttgtggc agaaagtcca    600
   aaaaaacct                                                            609
<210> 12
   <211> 609
   <212> DNA
   <213> Synthetic
<400> 12
   atgagccatc atcatcatca tcatcatcat tccatgggta ataattcagg tggggatact     60
   gcatctacta atcctgatga atctgttaag gggcctaatc ttacagaaat aagcaaaaaa    120
   attacagatt ctaatgcatt tgtactggct gtgaaagaag ttgaggcttt gatctcatct    180
   atagatgaac ttgctaaagc tattggtcaa agaatacaac aaaatggttt agttgctgat    240
   gcgggtcaca acagcgcatt gttagcagga gcccatgaaa tatcaatcct aataacacaa    300
   aaattagatg gattaaaagg tttagaagga ttaaaagcag agattgcaga agctaagaaa    360
   tattctgaag catttactaa aaaactaaaa gataatcatg cacagcttgg tatacagaat    420
   ggtgcttctc ttgatgatga ggcaaaaaaa gctattttaa aaacaaatgt ggacaaaacc    480
   aagggtgctg aagagcttga aaagttattt aaatcagtag aaagcttgtc aaaagcagcg    540
   caagaagcac taactaattc agttaaagag cttacaaatc ctgttgtggc agaaactcca    600
   aaaaaacct                                                            609
<210> 13
   <211> 609
   <212> DNA
   <213> Synthetic
<400> 13
   atgagccatc atcatcatca tcatcatcat tccatgtgta ataattcagg tggggatact      60
   gcatctacta atcctgatga atctgttaag gggcctaatc ttacagaaat aagcaaaaaa     120
   attacagatt ctaatgcatt tgtactggct gtgaaagaag ttgaggcttt gatctcatct     180
   atagatgaac ttgctaaagc tattggtcaa agaatacaac aaaatggttt agttgctgat     240
   gcgggtcaca acagcgcatt gttagcagga gcccatgaaa tatcaatcct aataacacaa     300
   aaattagatg gattaaaagg tttagaagga ttaaaagcag agattgcaga agctaagaaa     360
   tattctgaag catttactaa aaaactaaaa gataatcatg cacagcttgg tatacagaat     420
   ggtgcttctc ttgatgatga ggcaaaaaaa gctattttaa aaacaaatgt ggacaaaacc     480
   aagggtgctg aagagcttga aaagttattt aaatcagtag aaagcttgtc aaaagcagcg     540
   caagaagcac taactaattc agttaaagag cttacaaatc ctgttgtggc agaaactcca     600
   aaaaaacct                                                             609
<210> 14
   <211> 612
   <212> DNA
   <213> Synthetic
<400> 14
   atgagccatc atcatcatca tcatcatcat tccatgggta ataattcagg gaaagatggg      60
   aatacatctg caaattctgc tgatgagtct gttaaagggc ctaatcttac agaaataagt     120
   aaaaaaatta cggattctaa tgcggtttta cttgctgtga aagaggttga agcgttgctg     180
   tcatctatag atgaaattgc tgctaaagct attggtaaaa aaatacacca aaataatggt     240
   ttggataccg aaaataatca caatggatca ttgttagcgg gagcttatgc aatatcaacc     300
   ctaataaaac aaaaattaga tggattgaaa aatgaaggat taaaggaaaa aattgatgcg     360
   gctaagaaat gttctgaaac atttactaat aaattaaaag aaaaacacac agatcttggt     420
   aaagaaggtg ttactgatgc tgatgcaaaa gaagccattt taaaaacaaa tggtactaaa     480
   actaaaggtg ctgaagaact tggaaaatta tttgaatcag tagaggtctt gtcaaaagca     540
   gctaaagaga tgcttgctaa ttcagttaaa gagcttacaa gccctgttgt ggcagaaagt     600
   ccaaaaaaac ct                                                         612
<210> 15
   <211> 612
   <212> DNA
   <213> Synthetic
<400> 15
   atgagccatc atcatcatca tcatcatcat tccatgtgta ataattcagg gaaagatggg      60
   aatacatctg caaattctgc tgatgagtct gttaaagggc ctaatcttac agaaataagt     120
   aaaaaaatta cggattctaa tgcggtttta cttgctgtga aagaggttga agcgttgctg     180
   tcatctatag atgaaattgc tgctaaagct attggtaaaa aaatacacca aaataatggt     240
   ttggataccg aaaataatca caatggatca ttgttagcgg gagcttatgc aatatcaacc     300
   ctaataaaac aaaaattaga tggattgaaa aatgaaggat taaaggaaaa aattgatgcg     360
   gctaagaaat gttctgaaac atttactaat aaattaaaag aaaaacacac agatcttggt     420
   aaagaaggtg ttactgatgc tgatgcaaaa gaagccattt taaaaacaaa tggtactaaa     480
   actaaaggtg ctgaagaact tggaaaatta tttgaatcag tagaggtctt gtcaaaagca     540
   gctaaagaga tgcttgctaa ttcagttaaa gagcttacaa gccctgttgt ggcagaaagt     600
   ccaaaaaaac ct                                                         612
<210> 16
   <211> 31
   <212> DNA
   <213> Synthetic
<400> 16
   tagaccatgg gtaataattc agggaaaggt g 31
<210> 17
   <211> 42
   <212> DNA
   <213> Synthetic
<400> 17
   gcagagtgcc tcgagttaag gtttttttgg actttctgcc ac 42
<210> 18
   <211> 43
   <212> DNA
   <213> Synthetic
<400> 18
   atatcgtctc ccatgtgcaa taattcaggg aaaggtgggg ata 43
<210> 19
   <211> 44
   <212> DNA
   <213> Synthetic
<400> 19
   atatcgtctc ccatgggtaa taattcaggt ggggatactg catc 44
<210> 20
   <211> 36
   <212> DNA
   <213> Synthetic
<400> 20
   atactcgagt taaggttttt ttggagtttc tgccac 36
<210> 21
   <211> 44
   <212> DNA
   <213> Synthetic
<400> 21
   atatcgtctc ccatgtgtaa taattcaggt ggggatactg catc 44
<210> 22
   <211> 33
   <212> DNA
   <213> Synthetic
<400> 22
   tagaccatgg gtaataattc agggaaagat ggg 33
<210> 23
   <211> 44
   <212> DNA
   <213> Synthetic
<400> 23
   atatcgtctc ccatgtgcaa taattcaggg aaagatggga atac 44
<210> 24
   <211> 5287
   <212> DNA
   <213> Synthetic
<400> 24
   ggggaattgt gagcggataa caattcccct ctagaaataa ttttgtttaa ctttaagaag     60
   gagatatacc atgagccatc atcatcatca tcatcatcat tccatggcga tatccctcga    120
   gtaagtcgag caccaccacc accaccactg agatccggct gctaacaaag cccgaaagga    180
   agctgagttg gctgctgcca ccgctgagca ataactagca taaccccttg gggcctctaa    240
   acgggtcttg aggggttttt tgctgaaagg aggaactata tccggattgg cgaatgggac    300
   gcgccctgta gcggcgcatt aagcgcggcg ggtgtggtgg ttacgcgcag cgtgaccgct    360
   acacttgcca gcgccctagc gcccgctcct ttcgctttct tcccttcctt tctcgccacg    420
   ttcgccggct ttccccgtca agctctaaat cgggggctcc ctttagggtt ccgatttagt    480
   gctttacggc acctcgaccc caaaaaactt gattagggtg atggttcacg tagtgggcca    540
   tcgccctgat agacggtttt tcgccctttg acgttggagt ccacgttctt taatagtgga    600
   ctcttgttcc aaactggaac aacactcaac cctatctcgg tctattcttt tgatttataa    660
   gggattttgc cgatttcggc ctattggtta aaaaatgagc tgatttaaca aaaatttaac    720
   gcgaatttta acaaaatatt aacgtttaca atttcaggtg gcacttttcg gggaaatgtg    780
   cgcggaaccc ctatttgttt atttttctaa atacattcaa atatgtatcc gctcatgaat    840
   taattcttag aaaaactcat cgagcatcaa atgaaactgc aatttattca tatcaggatt    900
   atcaatacca tatttttgaa aaagccgttt ctgtaatgaa ggagaaaact caccgaggca    960
   gttccatagg atggcaagat cctggtatcg gtctgcgatt ccgactcgtc caacatcaat   1020
   acaacctatt aatttcccct cgtcaaaaat aaggttatca agtgagaaat caccatgagt   1080
   gacgactgaa tccggtgaga atggcaaaag tttatgcatt tctttccaga cttgttcaac   1140
   aggccagcca ttacgctcgt catcaaaatc actcgcatca accaaaccgt tattcattcg   1200
   tgattgcgcc tgagcgagac gaaatacgcg atcgctgtta aaaggacaat tacaaacagg   1260
   aatcgaatgc aaccggcgca ggaacactgc cagcgcatca acaatatttt cacctgaatc   1320
   aggatattct tctaatacct ggaatgctgt tttcccgggg atcgcagtgg tgagtaacca   1380
   tgcatcatca ggagtacgga taaaatgctt gatggtcgga agaggcataa attccgtcag   1440
   ccagtttagt ctgaccatct catctgtaac atcattggca acgctacctt tgccatgttt   1500
   cagaaacaac tctggcgcat cgggcttccc atacaatcga tagattgtcg cacctgattg   1560
   cccgacatta tcgcgagccc atttataccc atataaatca gcatccatgt tggaatttaa   1620
   tcgcggccta gagcaagacg tttcccgttg aatatggctc ataacacccc ttgtattact   1680
   gtttatgtaa gcagacagtt ttattgttca tgaccaaaat cccttaacgt gagttttcgt   1740
   tccactgagc gtcagacccc gtagaaaaga tcaaaggatc ttcttgagat cctttttttc   1800
   tgcgcgtaat ctgctgcttg caaacaaaaa aaccaccgct accagcggtg gtttgtttgc   1860
   cggatcaaga gctaccaact ctttttccga aggtaactgg cttcagcaga gcgcagatac   1920
   caaatactgt ccttctagtg tagccgtagt taggccacca cttcaagaac tctgtagcac   1980
   cgcctacata cctcgctctg ctaatcctgt taccagtggc tgctgccagt ggcgataagt   2040
   cgtgtcttac cgggttggac tcaagacgat agttaccgga taaggcgcag cggtcgggct   2100
   gaacgggggg ttcgtgcaca cagcccagct tggagcgaac gacctacacc gaactgagat   2160
   acctacagcg tgagctatga gaaagcgcca cgcttcccga agggagaaag gcggacaggt   2220
   atccggtaag cggcagggtc ggaacaggag agcgcacgag ggagcttcca gggggaaacg   2280
   cctggtatct ttatagtcct gtcgggtttc gccacctctg acttgagcgt cgatttttgt   2340
   gatgctcgtc aggggggcgg agcctatgga aaaacgccag caacgcggcc tttttacggt   2400
   tcctggcctt ttgctggcct tttgctcaca tgttctttcc tgcgttatcc cctgattctg   2460
   tggataaccg tattaccgcc tttgagtgag ctgataccgc tcgccgcagc cgaacgaccg   2520
   agcgcagcga gtcagtgagc gaggaagcgg aagagcgcct gatgcggtat tttctcctta   2580
   cgcatctgtg cggtatttca caccgcatat atggtgcact ctcagtacaa tctgctctga   2640
   tgccgcatag ttaagccagt atacactccg ctatcgctac gtgactgggt catggctgcg   2700
   ccccgacacc cgccaacacc cgctgacgcg ccctgacggg cttgtctgct cccggcatcc   2760
   gcttacagac aagctgtgac cgtctccggg agctgcatgt gtcagaggtt ttcaccgtca   2820
   tcaccgaaac gcgcgaggca gctgcggtaa agctcatcag cgtggtcgtg aagcgattca   2880
   cagatgtctg cctgttcatc cgcgtccagc tcgttgagtt tctccagaag cgttaatgtc   2940
   tggcttctga taaagcgggc catgttaagg gcggtttttt cctgtttggt cactgatgcc   3000
   tccgtgtaag ggggatttct gttcatgggg gtaatgatac cgatgaaacg agagaggatg   3060
   ctcacgatac gggttactga tgatgaacat gcccggttac tggaacgttg tgagggtaaa   3120
   caactggcgg tatggatgcg gcgggaccag agaaaaatca ctcagggtca atgccagcgc   3180
   ttcgttaata cagatgtagg tgttccacag ggtagccagc agcatcctgc gatgcagatc   3240
   cggaacataa tggtgcaggg cgctgacttc cgcgtttcca gactttacga aacacggaaa   3300
   ccgaagacca ttcatgttgt tgctcaggtc gcagacgttt tgcagcagca gtcgcttcac   3360
   gttcgctcgc gtatcggtga ttcattctgc taaccagtaa ggcaaccccg ccagcctagc   3420
   cgggtcctca acgacaggag cacgatcatg cgcacccgtg gggccgccat gccggcgata    3480
   atggcctgct tctcgccgaa acgtttggtg gcgggaccag tgacgaaggc ttgagcgagg   3540
   gcgtgcaaga ttccgaatac cgcaagcgac aggccgatca tcgtcgcgct ccagcgaaag   3600
   cggtcctcgc cgaaaatgac ccagagcgct gccggcacct gtcctacgag ttgcatgata   3660
   aagaagacag tcataagtgc ggcgacgata gtcatgcccc gcgcccaccg gaaggagctg   3720
   actgggttga aggctctcaa gggcatcggt cgagatcccg gtgcctaatg agtgagctaa   3780
   cttacattaa ttgcgttgcg ctcactgccc gctttccagt cgggaaacct gtcgtgccag   3840
   ctgcattaat gaatcggcca acgcgcgggg agaggcggtt tgcgtattgg gcgccagggt   3900
   ggtttttctt ttcaccagtg agacgggcaa cagctgattg cccttcaccg cctggccctg   3960
   agagagttgc agcaagcggt ccacgctggt ttgccccagc aggcgaaaat cctgtttgat   4020
   ggtggttaac ggcgggatat aacatgagct gtcttcggta tcgtcgtatc ccactaccga   4080
   gatatccgca ccaacgcgca gcccggactc ggtaatggcg cgcattgcgc ccagcgccat   4140
   ctgatcgttg gcaaccagca tcgcagtggg aacgatgccc tcattcagca tttgcatggt   4200
   ttgttgaaaa ccggacatgg cactccagtc gccttcccgt tccgctatcg gctgaatttg   4260
   attgcgagtg agatatttat gccagccagc cagacgcaga cgcgccgaga cagaacttaa   4320
   tgggcccgct aacagcgcga tttgctggtg acccaatgcg accagatgct ccacgcccag   4380
   tcgcgtaccg tcttcatggg agaaaataat actgttgatg ggtgtctggt cagagacatc   4440
   aagaaataac gccggaacat tagtgcaggc agcttccaca gcaatggcat cctggtcatc   4500
   cagcggatag ttaatgatca gcccactgac gcgttgcgcg agaagattgt gcaccgccgc   4560
   tttacaggct tcgacgccgc ttcgttctac catcgacacc accacgctgg cacccagttg   4620
   atcggcgcga gatttaatcg ccgcgacaat ttgcgacggc gcgtgcaggg ccagactgga   4680
   ggtggcaacg ccaatcagca acgactgttt gcccgccagt tgttgtgcca cgcggttggg   4740
   aatgtaattc agctccgcca tcgccgcttc cactttttcc cgcgttttcg cagaaacgtg   4800
   gctggcctgg ttcaccacgc gggaaacggt ctgataagag acaccggcat actctgcgac   4860
   atcgtataac gttactggtt tcacattcac caccctgaat tgactctctt ccgggcgcta   4920
   tcatgccata ccgcgaaagg ttttgcgcca ttcgatggtg tccgggatct cgacgctctc   4980
   ccttatgcga ctcctgcatt aggaagcagc ccagtagtag gttgaggccg ttgagcaccg   5040
   ccgccgcaag gaatggtgca tgcaaggaga tggcgcccaa cagtcccccg gccacggggc   5100
   ctgccaccat acccacgccg aaacaagcgc tcatgagccc gaagtggcga gcccgatctt   5160
   ccccatcggt gatgtcggcg atataggcgc cagcaaccgc acctgtggcg ccggtgatgc   5220
   cggccacgat gcgtccggcg tagaggatcg agatctcgat cccgcgaaat taatacgact   5280
   cactata                                                            5287